# EUROPEAN PATENT SPECIFICATION

(11) **EP 3 660 935 B1**
(45) Date of publication and mention of the grant of the patent: **19.08.2026**
(21) Application number: 19204007.9
(22) Date of filing: 18.10.2019
(51) Int. Cl.: H10K 30/30, H10K 85/60, C07D 495/22, C07D 517/22, H10K 30/40, H10K 39/32

(54) **PHOTOELECTRIC CONVERSION DEVICES AND ORGANIC SENSORS AND ELECTRONIC DEVICES**
PHOTOELEKTRISCHE UMWANDLUNGSVORRICHTUNGEN UND ORGANISCHE SENSOREN UND ELEKTRONISCHE VORRICHTUNGEN
DISPOSITIFS DE CONVERSION PHOTOÉLECTRIQUE ET CAPTEURS ORGANIQUES ET DISPOSITIFS ÉLECTRONIQUES

(30) Priority: 14.11.2018 KR 20180139826; 17.10.2019 KR 20190129094
(43) Date of publication of application: 03.06.2020
(73) Proprietor: Samsung Electronics Co., Ltd., Gyeonggi-do 16677 (KR)
(72) Inventor: HEO, Chul Joon, Busan (KR); PARK, Kyung Bae, Gyeonggi-do (KR); KANG, Hyun Bum, Gyeonggi-do (KR); PARK, Sung Jun, Gyeonggi-do (KR); PARK, Jeong Il, Gyeonggi-do (KR); BAIK, Chul, Gyeonggi-do (KR); SHIN, Ji Soo, Seoul (KR); YUN, Sung Young, Gyeonggi-do (KR); LEE, Gae Hwang, Gyeonggi-do (KR); LEE, Don-Wook, Seoul (KR); LEE, Eun Kyung, Seoul (KR); JIN, Yong Wan, 01387 Seoul (KR); CHOI, Yeong Suk, Gyeonggi-do (KR); CHOI, Taejin, Gyeonggi-do (KR)
(74) Representative: Elkington and Fife LLP

(56) References cited:
- EP-A1- 3 300 114
- EP-A1- 3 306 689
- EP-A2- 2 259 358
- EP-A2- 2 448 031
- WO-A1-2010/095517
- KR-A- 20160 094 704
- US-A1- 2016 226 005
- US-A1- 2017 294 593
- US-B2- 9 455 059
- KE YANG ET AL: "Determination of Electron and Hole Mobility of Regioregular Poly(3-hexylthiophene) by the Time of Flight Method", JOURNAL OF MACROMOLECULAR SCIENCE , PART A - PURE AND APPLIED CHEMISTRY., vol. 44, no. 12, 1 October 2007 (2007-10-01), US, pages 1261 - 1264, XP055677022, ISSN: 1060-1325, DOI: 10.1080/10601320701606711
- HONGZE GAO ET AL: "Theoretical Characterization of a Typical Hole/Exciton-Blocking Material Bathocuproine and Its Analogues", JOURNAL OF PHYSICAL CHEMISTRY. A, MOLECULES, SPECTROSCOPY,KINETICS, ENVIRONMENT AND GENERAL THEORY, vol. 112, no. 38, 27 August 2008 (2008-08-27), US, pages 9097 - 9103, XP055677162, ISSN: 1089-5639, DOI: 10.1021/jp804308e
- S. A. RUTLEDGE ET AL: "Carrier mobility enhancement in poly(3,4-ethylenedioxythiophene)-poly(styrenesulfonate) having undergone rapid thermal annealing", JOURNAL OF APPLIED PHYSICS, vol. 114, no. 13, 7 October 2013 (2013-10-07), US, pages 133708, XP055676738, ISSN: 0021-8979, DOI: 10.1063/1.4824104
- ISHIYAMA NORIHIRO ET AL: "Tandem organic solar cells formed in co-deposited films by doping", ORGANIC ELECTRONICS, ELSEVIER, AMSTERDAM, NL, vol. 14, no. 7, 15 April 2013 (2013-04-15), pages 1793 - 1796, XP028554612, ISSN: 1566-1199, DOI: 10.1016/J.ORGEL.2013.04.003

## Description

### FIELD OF THE INVENTION

Photoelectric conversion devices, organic sensors, and electronic devices are disclosed.

### BACKGROUND OF THE INVENTION

A photoelectric conversion device may receive incident light and convert the received incident light into an electric signal. A photoelectric conversion device may include a photodiode and a phototransistor, and may be applied to ("included in") an organic sensor, a photodetector, a solar cell, or the like.

Organic sensors may have higher resolutions and thus may have smaller pixel sizes. Organic sensors may include silicon photodiodes. A sensitivity of a silicon photodiode in an organic sensor may be deteriorated based on reduced pixel size of the organic sensor, as the absorption area of the silicon photodiode may be reduced. Accordingly, organic materials that are capable of replacing silicon in photodiodes of organic sensors have been researched.

An organic material has a high extinction coefficient and is configured to selectively absorb light in a particular wavelength spectrum of light depending on a molecular structure of the organic material, and thus may simultaneously replace a photodiode and a color filter of an organic sensor and resultantly improve sensitivity of the organic sensor and contribute to high integration of the organic sensor.

However, since such organic materials exhibit different characteristics from those of silicon due to high binding energy and a recombination behavior associated with such organic materials, the characteristics of the organic materials are difficult to precisely predict, and thus required properties of a photoelectric conversion device may not be easily controlled.

EP 2 448 031 A2 discloses a photoelectric conversion device having: a pair of electrodes; a photoelectric conversion layer sandwiched between the pair of electrodes; and at least one electron blocking layer provided between one electrode of the pair of electrodes and the photoelectric conversion later.

Ishiyama et al. have disclosed (Org. Electron. 2013, 14, 1793-1796) tandem organic solar cells, in which two single *p*⁺*in*⁺-homojunctions are connected by a heavily doped *n*⁺*p*⁺-ohmic interlayer.

### SUMMARY OF THE INVENTION

Some example embodiments provide one or more photoelectric conversion devices capable of improving charge extraction efficiency.

Some example embodiments provide organic sensors including one or more of the photoelectric conversion devices.

Some example embodiments provide electronic devices including the one or more photoelectric conversion devices or the one or more organic sensors.

According to the invention, a photoelectric conversion device is provided in accordance with claim 1.

The charge mobility of the organic auxiliary layer may be at least about 100 times as high as the charge mobility of the photoelectric conversion layer.

The charge mobility of the organic auxiliary layer may be greater than or equal to about 1.0x10⁻³ cm²/Vs.

The electron mobility of a second organic material included in the photoelectric conversion device may be greater than or equal to 1.0x10⁻³ cm²/Vs.

A semiconductor included in the photoelectric conversion device may include fullerene or a fullerene derivative.

A thickness of the organic auxiliary layer may be less than or equal to 5 nm.

The photoelectric conversion device may further include an inorganic nanolayer between the first electrode and the photoelectric conversion layer.

The inorganic nanolayer may include a lanthanide element, calcium (Ca), potassium (K), aluminum (Al), or an alloy thereof.

The lanthanide element may include ytterbium (Yb).

A thickness of the inorganic nanolayer may be less than or equal to 5 nm.

The organic auxiliary layer may be in contact with the photoelectric conversion layer and the inorganic nanolayer may be in contact with the first electrode.

An electronic device may include the photoelectric conversion device.

An organic sensor may include the photoelectric conversion device.

An electronic device may include the organic sensor.

The photoelectric conversion device may further include an inorganic nanolayer between the first electrode and the photoelectric conversion layer.

By improving the charge mobility, residual charges may be reduced and thus charge extraction efficiency of the photoelectric conversion device may be increased.

### BRIEF DESCRIPTION OF THE DRAWINGS

FIG. 1 is a cross-sectional view showing an example of a photoelectric conversion device according to some example embodiments,
FIG. 2 is a cross-sectional view showing an example of a photoelectric conversion device according to some example embodiments,
FIG. 3 is a cross-sectional view showing an example of a photoelectric conversion device according to some example embodiments,
FIG. 4 is a cross-sectional view showing an example of a photoelectric conversion device according to some example embodiments,
FIG. 5 is a schematic cross-sectional view showing an example of an organic sensor according to some example embodiments,
FIG. 6 is a schematic cross-sectional view showing an example of an organic sensor according to some example embodiments,
FIG. 7 is a schematic top plan view showing an example of an organic sensor according to some example embodiments,
FIG. 8 is a schematic cross-sectional view showing an example of the organic sensor of FIG. 7,
FIG. 9 is a schematic cross-sectional view showing an example of the organic sensor of FIG. 7,
FIG. 10 is a schematic cross-sectional view showing an example of an organic sensor according to some example embodiments,
FIG. 11 is a schematic cross-sectional view showing an example of an organic sensor according to some example embodiments,
FIG. 12 is a schematic top plan view showing an example of an organic sensor according to some example embodiments,
FIG. 13 is a schematic cross-sectional view showing an example of the organic sensor of FIG. 12,
FIG. 14 is a schematic cross-sectional view showing an example of the organic sensor of FIG. 12,
FIG. 15 is a schematic top plan view showing an example of an organic sensor according to some example embodiments, and FIG. 16 is a schematic cross-sectional view showing an example of the organic sensor of FIG. 15,
FIG. 17 is a schematic cross-sectional view of an organic sensor according to some example embodiments,
FIG. 18 is a schematic cross-sectional view of an organic sensor according to some example embodiments, and
FIG. 19 is a schematic diagram of an electronic device according to some example embodiments.

### DETAILED DESCRIPTION OF THE EMBODIMENTS

Hereinafter, example embodiments of the present disclosure will be described in detail so that a person skilled in the art would understand the same. However, this disclosure may be embodied in many different forms and is not to be construed as limited to the example embodiments set forth herein.

In the drawings, the thickness of layers, films, panels, regions, etc., are exaggerated for clarity.

It will be understood that when an element such as a layer, film, region, or substrate is referred to as being "on" another element, it may be directly on the other element or intervening elements may also be present. In contrast, when an element is referred to as being "directly on" another element, there are no intervening elements present.

As used herein, when a definition is not otherwise provided, "substituted" may refer to replacement of a hydrogen atom of a compound or a group by a substituent selected from a halogen atom, a hydroxy group, an alkoxy group, a nitro group, a cyano group, an amino group, an azido group, an amidino group, a hydrazino group, a hydrazono group, a carbonyl group, a carbamyl group, a thiol group, an ester group, a carboxyl group or a salt thereof, a sulfonic acid group or a salt thereof, phosphoric acid group or a salt thereof, silyl group, a C1 to C20 alkyl group, a C2 to C20 alkenyl group, a C2 to C20 alkynyl group, a C6 to C30 aryl group, a C7 to C30 arylalkyl group, a C1 to C30 alkoxy group, a C1 to C20 heteroalkyl group, a C3 to C20 heteroaryl group, a C3 to C20 heteroarylalkyl group, a C3 to C30 cycloalkyl group, a C3 to C15 cycloalkenyl group, a C6 to C15 cycloalkynyl group, a C3 to C30 heterocycloalkyl group, and a combination thereof.

As used herein, when a definition is not otherwise provided, "hetero" may refer to one including 1 to 4 heteroatoms selected from N, O, S, Se, Te, Si, and P.

As used herein, when a definition is not otherwise provided, "heterocyclic group" is a generic concept of a heteroaryl group, may include an aromatic and nonaromatic ring including at least one heteroatom, and may include at least one heteroatom selected from N, O, S, P, and Si instead of carbon (C) in a cyclic group such as an aryl group, a cycloalkyl group, a fused ring thereof, or a combination thereof. When the heterocyclic group is a fused ring, the entire ring or each ring of the heterocyclic group may include one or more heteroatoms.

Hereinafter, "combination" may refer to a mixture of two or more and a stack structure of two or more.

Hereinafter, "metal" may refer to metal, semi-metal, or a combination thereof.

Hereinafter, a work function or an energy level is expressed as an absolute value from a vacuum level. In addition, when the work function or the energy level is referred to be deep, high, or large, it may have a large absolute value from "0 eV" of the vacuum level while when the work function or the energy level is referred to be shallow, low, or small, it may have a small absolute value from "0 eV" of the vacuum level.

Hereinafter, the charge mobility may be a value estimated in a diode structure.

Hereinafter, a photoelectric conversion device according to some example embodiments is described.

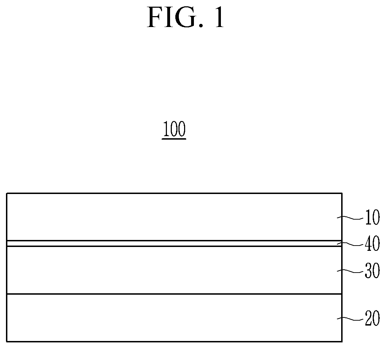

FIG. 1 is a cross-sectional view showing an example of a photoelectric conversion device according to some example embodiments.

Referring to FIG. 1, a photoelectric conversion device 100 according to the invention includes a first electrode 10, a second electrode 20, a photoelectric conversion layer 30, and an organic auxiliary layer 40.

A substrate (not shown) may be disposed at the side of the first electrode 10 or the second electrode 20. The substrate may be for example made of an inorganic material such as glass, an organic material such as polycarbonate, polymethylmethacrylate, polyethyleneterephthalate, polyethylenenaphthalate, polyamide, polyethersulfone, or a combination thereof, or a silicon wafer. The substrate may be omitted.

As shown, the first electrode 10 and the second electrode 20 are facing each other. One of the first electrode 10 and the second electrode 20 is an anode and the other is a cathode. The first electrode 10 is a cathode and the second electrode 20 is an anode.

At least one of the first electrode 10 and the second electrode 20 may be a transparent electrode, such that the at least one of the first electrode 10 and the second electrode 20 includes a transparent conductor. Herein, the transparent electrode may include a transparent conductor having a high light transmittance of greater than or equal to about 80 % and may not include for example a semitransparent electrode for microcavity. The transparent electrode may include for example a transparent conductor that includes at least one of an oxide conductor and a carbon conductor. The oxide conductor may include for example at least one of indium tin oxide (ITO), indium zinc oxide (IZO), zinc tin oxide (ZTO), aluminum tin oxide (AlTO), and aluminum zinc oxide (AZO) and the carbon conductor may at least one of graphene and carbon nanostructure.

One of the first electrode 10 and the second electrode 20 may be a reflective electrode, such that the at least one of the first electrode 10 and the second electrode 20 includes a reflective conductor. Here, the reflective electrode may include, in some example embodiments, a reflective conductor having a light transmittance of less than about 10 % or high reflectance of more than or equal to about 5 %. The reflective electrode may include a reflective conductor such as a metal and may include, for example aluminum (Al), silver (Ag), gold (Au), or an alloy thereof.

In some example embodiments, the first electrode 10 may be a transparent electrode (e.g., the first electrode 10 may include a transparent conductor) having a light transmittance of greater than or equal to about 80 % or a reflective electrode having a light transmittance of less than about 10 %.

In view of the above, it will be understood that the first electrode 10 and/or the second electrode 20 may include a conductor, where the conductor may be a transparent conductor or a reflective conductor. A conductor that is a transparent conductor may have a light transmittance of greater than or equal to about 80 %. A conductor that is a reflective conductor may have a light transmittance of less than about 10 %.

The photoelectric conversion layer 30, which, as shown, is between the first electrode 10 and the second electrode 20, is configured to absorb light in at least one part of a wavelength spectrum of light and convert the absorbed light into an electric signal, and for example one of light in a green wavelength spectrum of light (hereinafter, referred to as "green light"), light in a blue wavelength spectrum of light (hereinafter, referred to as "blue light"), light in a red wavelength spectrum of light (hereinafter, referred to as "red light"), light in an ultraviolet wavelength spectrum of light (hereinafter, referred to as 'ultraviolet light'), and light in an infrared wavelength spectrum of light (hereinafter, referred to as 'infrared light') into an electric signal.

In some example embodiments, the photoelectric conversion layer 30 may be configured to selectively absorb at least one of the green light, the blue light, the red light, and the infrared light. Herein, the selective absorption of at least one from the green light, the blue light, the red light, and the infrared light means that a light-absorption spectrum has a peak absorption wavelength (λₘₐₓ) in one of about 500 nm to about 600 nm, greater than or equal to about 380 nm and less than about 500 nm, greater than about 600 nm and less than or equal to about 700 nm, and greater than about 700 nm, and a light-absorption spectrum in the corresponding wavelength spectrum of light is remarkably higher than those in the other wavelength spectra of light.

The photoelectric conversion layer 30 includes a first organic material which is a p-type semiconductor represented by Chemical Formula 1A and an n-type semiconductor material that forms a pn junction with the first organic material of the photoelectric conversion layer 30 and may produce excitons by receiving light from outside and then separate the produced excitons into holes and electrons.

The p-type semiconductor and the n-type semiconductor may be independently light-absorbing materials, and for example at least one of the p-type semiconductor and the n-type semiconductor may be an organic light-absorbing material. In some example embodiments, at least one of the p-type semiconductor and the n-type semiconductor may be a wavelength-selective light-absorbing material that selectively absorbs light in a particular (or, alternatively, predetermined) wavelength spectrum of light, and for example at least one of the p-type semiconductor and the n-type semiconductor may be a wavelength-selective organic light-absorbing material. The p-type semiconductor and the n-type semiconductor may have a peak absorption wavelength (λₘₐₓ) in the same wavelength spectrum of light or in a different wavelength spectrum of light.

The p-type semiconductor is represented by Chemical Formula 1A.

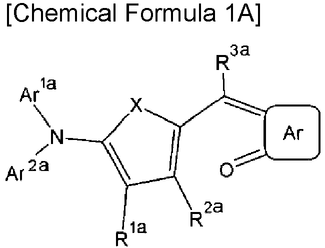

In Chemical Formula 1A,
X is S, Se, Te, SO, SO₂, or SiR^{a}R^{b},
Ar is a substituted or unsubstituted C6 to C30 arylene group, a substituted or unsubstituted C3 to C30 heterocyclic group, or a fused ring of the foregoing two or more,
Ar^{1a} and Ar^{2a} are independently a substituted or unsubstituted C6 to C30 aryl group or a substituted or unsubstituted C3 to C30 heteroaryl group,
Ar^{1a} and Ar^{2a} are independently present alone or may be linked with each other to form a fused ring, and
R^{1a} to R^{3a}, R^{a}, and R^{b} are independently hydrogen, deuterium, a substituted or unsubstituted C1 to C30 alkyl group, a substituted or unsubstituted C6 to C30 aryl group, a substituted or unsubstituted C3 to C30 heteroaryl group, a substituted or unsubstituted C1 to C6 alkoxy group, a halogen, or a cyano group.

In some example embodiments, in Chemical Formula 1A, Ar1a and Ar2a may independently be one of a substituted or unsubstituted phenyl group, a substituted or unsubstituted naphthyl group, a substituted or unsubstituted anthracenyl group, a substituted or unsubstituted phenanthrenyl group, a substituted or unsubstituted pyridinyl group, a substituted or unsubstituted pyridazinyl group, a substituted or unsubstituted pyrimidinyl group, a substituted or unsubstituted pyrazinyl group, a substituted or unsubstituted quinolinyl group, a substituted or unsubstituted isoquinolinyl group, a substituted or unsubstituted naphthyridinyl group, a substituted or unsubstituted cinnolinyl group, a substituted or unsubstituted quinazolinyl group, a substituted or unsubstituted phthalazinyl group, a substituted or unsubstituted benzotriazinyl group, a substituted or unsubstituted pyridopyrazinyl group, a substituted or unsubstituted pyridopyrimidinyl group, and a substituted or unsubstituted pyridopyridazinyl group.

In some example embodiments, Ar^{1a} and Ar^{2a} of Chemical Formula 1A may be linked with each other to form a ring or in some example embodiments, Ar^{1a} and Ar^{2a} may be linked with each other by one of a single bond, -(CR^{g}R^{h})ₙ₂- (n2 is 1 or 2), -O-, -S-, -Se-, -N=, -NRⁱ-, -SiRⁱR^{k}-, and -GeR^{l}R^{m}- to form a ring. Herein, R^{g} to R^{m} may independently be hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C1 to C6 alkoxy group, a halogen, or a cyano group.

The n-type semiconductor may be for example fullerene or a fullerene derivative, but is not limited thereto.

The photoelectric conversion layer 30 is an intrinsic layer (an I layer) wherein the p-type semiconductor and the n-type semiconductor are mixed as a bulk heterojunction. Herein, the p-type semiconductor and the n-type semiconductor may be mixed in a volume ratio of about 1:9 to about 9:1, for example about 2:8 to about 8:2, about 3:7 to about 7:3, about 4:6 to about 6:4, or about 5:5.

The photoelectric conversion layer 30 may be a bilayer including a p-type layer including the p-type semiconductor and an n-type layer including the n-type semiconductor. Herein, a thickness ratio of the p-type layer and the n-type layer may be about 1:9 to about 9:1, for example about 2:8 to about 8:2, about 3:7 to about 7:3, about 4:6 to about 6:4, or about 5:5.

The photoelectric conversion layer 30 may further include a p-type layer and/or an n-type layer in addition to the intrinsic layer. The p-type layer may include the p-type semiconductor and the n-type layer may include the n-type semiconductor. In some example embodiments, they may be included in various combinations of p-type layer/l layer, I layer/n-type layer, p-type layer/l layer/n-type layer, and the like.

The organic auxiliary layer 40 is disposed between the first electrode 10 and the photoelectric conversion layer 30 and may be for example in contact with the photoelectric conversion layer 30. One surface of the organic auxiliary layer 40 may be in contact with the first electrode 10 and the other surface of the organic auxiliary layer 40 may be in contact with the photoelectric conversion layer 30. The organic auxiliary layer 40 includes the semiconductor material included in the photoelectric conversion layer, such that the organic auxiliary layer 40 includes the p-type semiconductor and/or the n-type semiconductor. It will be understood that, in some example embodiments, the organic auxiliary layer 40 may not be in contact with the first electrode 10.

The organic auxiliary layer 40 is a layer configured to effectively improve the extraction of charges (e.g., electrons) moving from the photoelectric conversion layer 30 to the first electrode 10, and may include for example an organic semiconductor having a high charge mobility.

The charge mobility of the organic auxiliary layer 40 is higher than the charge mobility of the photoelectric conversion layer 30. In some example embodiments, the charge mobility of the organic auxiliary layer 40 may be at least about 50 times, for example at least about 70 times, at least about 80 times, at least about 100 times, at least about 120 times, at least about 150 times, or at least about 200 times as high as the charge mobility of the photoelectric conversion layer 30.

When the terms "about" or "substantially" are used in this specification in connection with a numerical value, it is intended that the associated numerical value include a tolerance of ±10% around the stated numerical value. When ranges are specified, the range includes all values therebetween such as increments of 0.1%.

In some example embodiments, the charge mobility of the organic auxiliary layer 40 may be for example greater than or equal to about 1.0x10⁻³ cm²/Vs, greater than or equal to about 1.2x10⁻³ cm²/Vs, greater than or equal to about 1.5 x10⁻³ cm²/Vs, greater than or equal to about 1.8x10⁻³ cm²/Vs, greater than or equal to about 2.0x10⁻³ cm²/Vs, greater than or equal to about 3.0x10⁻³ cm²/Vs, greater than or equal to about 4.0x10⁻³ cm²/Vs, or greater than or equal to about 5.0x10⁻³ cm²/Vs.

In some example embodiments, the charge mobility of the organic auxiliary layer 40 may be for example about 1.0x10⁻³ cm²/Vs to about 10 cm²/Vs, about 1.2x10⁻³ cm²/Vs to about 10 cm²/Vs, about 1.5 x10⁻³ cm²/Vs to about 10 cm²/Vs, about 1.8x10⁻³ cm²/Vs to about 10 cm²/Vs, about 2.0 x10⁻³ cm²/Vs to about 10 cm²/Vs, about 3.0x10⁻³ cm²/Vs to about 10 cm²/Vs, about 4.0x10⁻³ cm²/Vs to about 10 cm²/Vs, or about 5.0x10³ cm²/Vs to about 10 cm²/Vs.

The photoelectric conversion layer 30 includes a first organic material that is the p-type semiconductor and the organic auxiliary layer 40 includes a second organic material that is different from the first organic material. The photoelectric conversion layer 30 includes a first organic material that is a p-type semiconductor and the organic auxiliary layer 40 includes a second organic material that is an n-type semiconductor. An electron mobility of the second organic material is at least 100 times as high as an electron mobility of the first organic material.

In some example embodiments, the electron mobility of the second organic material may be for example greater than or equal to 1.0x10⁻³ cm²/Vs, greater than or equal to about 1.2x10⁻³ cm²/Vs, greater than or equal to about 1.5 x10⁻³ cm²/Vs, greater than or equal to about 1.8x10⁻³ cm²/Vs, greater than or equal to about 2.0 x10⁻³ cm²/Vs, greater than or equal to about 3.0x10⁻³ cm²/Vs, greater than or equal to about 4.0x10⁻³ cm²/Vs, or greater than or equal to about 5.0x10⁻³ cm²/Vs.

In some example embodiments, the electron mobility of the second organic material may be for example 1.0x10⁻³ cm²/Vs to about 10 cm²/Vs, about 1.2x10⁻³ cm²/Vs to about 10 cm²/Vs, about 1.5 x10⁻³ cm²/Vs to about 10 cm²/Vs, about 1.8x10⁻³ cm²/Vs to about 10 cm²/Vs, about 2.0 x10⁻³ cm²/Vs to about 10 cm²/Vs, about 3.0x10⁻³ cm²/Vs to about 10 cm²/Vs, about 4.0x10⁻³ cm²/Vs to about 10 cm²/Vs, or about 5.0x10⁻³ cm²/Vs to about 10 cm²/Vs.

In some example embodiments, the second organic material may be a material which satisfies the above-described charge mobility and may be formed by thermal evaporation. Since the organic auxiliary layer 40 is formed by thermal evaporation as described above, it is possible to prevent the photoelectric conversion layer 30 from being thermally and physically damaged in its formation process and/or the subsequent process of the organic auxiliary layer 40, and thus to prevent its performance from being deteriorated due to degradation of the photoelectric conversion layer 30.

The second organic material that may satisfy such characteristics may be, in some example embodiments, a low molecular organic semiconductor, a polymer semiconductor, or a combination thereof, in some example embodiments, a low molecular organic semiconductor. Herein, the low molecular organic semiconductor may be an organic semiconductor having a weight average molecular weight of less than or equal to about 3000.

In some example embodiments, the second organic material may include a second organic material compound that may be an aromatic compound and/or a heteroaromatic compound, for example a fused polycyclic aromatic compound, a fused polycyclic heteroaromatic compound, or a combination thereof, for example a fused polycyclic aromatic compound such as pentacene and/or a fused polycyclic heteroaromatic compound including at least one of O, S, Se, Te, N, or a combination thereof, for example a fused polycyclic heteroaromatic compound including S, Se, Te, or a combination thereof.

In some example embodiments, the second organic material may include a second organic material compound that may be a fused polycyclic aromatic compound and/or fused polycyclic heteroaromatic compound having a compact planar structure wherein four or more rings are fused with each other, for example a fused polycyclic aromatic compound and/or a fused polycyclic heteroaromatic compound wherein 5, 6, 7, 8, 9, 10, 11, or 12 rings are condensed. Restated, the second organic material may include a second organic material compound that includes at least four rings that are fused.

In some example embodiments, the second organic material may be a fused polycyclic aromatic compound and/or a fused polycyclic heteroaromatic compound including at least one benzene ring.

In some example embodiments, the second organic material may be a fused polycyclic heteroaromatic compound including at least one thiophene, selenophene, and/or tellurophene.

The second organic material is represented by Chemical Formula 2A or 2B.

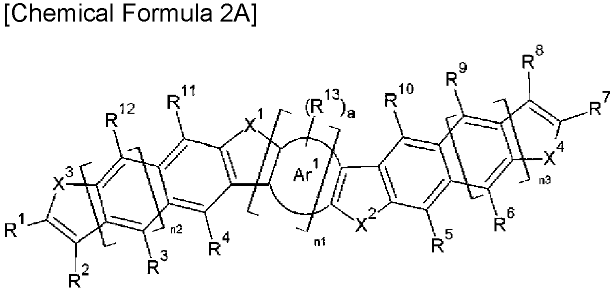

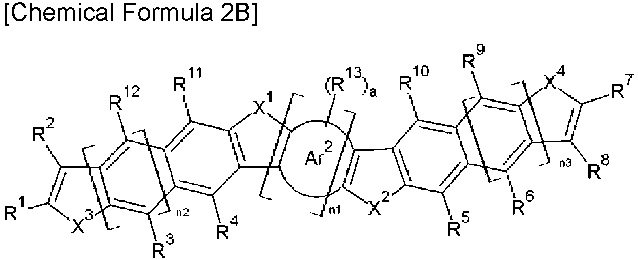

In Chemical Formulae 2A and 2B,
Ar¹ and Ar² are independently a substituted or unsubstituted benzene, a substituted or unsubstituted naphthalene, or a substituted or unsubstituted anthracene,
a corresponds to the number of hydrogen bound to carbon of Ar¹ and Ar²,

X¹ to X⁴ are independently O, S, Se, Te, or N-R^{a}, wherein R^{a} may independently be hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C7 to C30 arylalkyl group, a substituted or unsubstituted C6 to C30 aryl group, a substituted or unsubstituted C1 to C30 alkoxy group, a substituted or unsubstituted C6 to C30 aryloxy group (-OR^{b}, wherein R^{b} is a substituted or unsubstituted C6 to C30 aryl group), a substituted or unsubstituted C4 to C30 cycloalkyl group, a substituted or unsubstituted C4 to C30 cycloalkyloxy group (-OR^{c}, wherein R^{c} is a substituted or unsubstituted C4 to C30 cycloalkyl group), a substituted or unsubstituted C2 to C30 heteroaryl group, acyl group (-C(=O)R^{d}, wherein R^{d} is a substituted or unsubstituted C1 to C30 alkyl group), a sulfonyl group (-S(=O)₂R^{e}, wherein R^{e} is a substituted or unsubstituted C1 to C30 alkyl group), or carbamate group (-NHC(=O)OR^{f}, wherein R^{f} is a substituted or unsubstituted C1 to C30 alkyl group),
R¹ to R¹³ are independently hydrogen, a substituted or unsubstituted C1 to C30 alkyl group, a substituted or unsubstituted C1 to C30 alkoxy group, a substituted or unsubstituted C2 to C30 alkenyl group, a substituted or unsubstituted C2 to C30 alkynyl group, a substituted or unsubstituted C6 to C30 aryl group, a substituted or unsubstituted C2 to C30 heteroaryl group, a substituted or unsubstituted C7 to C30 arylalkyl group, a substituted or unsubstituted C2 to C30 heteroarylalkyl group, a substituted or unsubstituted C2 to C30 alkylheteroaryl group, a substituted or unsubstituted C5 to C30 cycloalkyl group, or a substituted or unsubstituted C2 to C30 heterocycloalkyl group,
   n1 is 0 or 1,
   n2 and n3 are independently 0, 1, 2, or 3,
   when n1 is 0, n2 and n3 is 1, 2, or 3, and
   when n1 is 1, n1+n2+n3≥2.

In some example embodiments, R¹ and R⁷ may be a substituted or unsubstituted C1 to C30 alkyl group, a substituted or unsubstituted C6 to C30 aryl group, a substituted or unsubstituted C2 to C30 heteroaryl group, a substituted or unsubstituted C7 to C30 arylalkyl group, a substituted or unsubstituted C2 to C30 heteroarylalkyl group, a substituted or unsubstituted C2 to C30 alkylheteroaryl group, a substituted or unsubstituted C5 to C30 cycloalkyl group, or a substituted or unsubstituted C2 to C30 heterocycloalkyl group.

In some example embodiments, R^{a} may be a substituted or unsubstituted C10 to C30 alkyl group, a substituted or unsubstituted C10 to C30 alkoxy group, a substituted or unsubstituted C10 to C30 alkenyl group, or a substituted or unsubstituted C10 to C30 alkynyl group, and for another example, a fluoro substituted C1 to C30 alkyl group, desirably a C1 to C30 perfluoro alkyl group (CₙF₂ₙ₊₁, wherein n is an integer of 1 to 30) or a fluoro substituted C10 to C30 alkyl group, desirably a C10 to C30 perfluoro alkyl group (CₙF₂ₙ₊₁, wherein n is an integer of 10 to 30).

In Chemical Formulae 2A and 2B, when n1 is 0, n2 and n3 may be an integer of 1, 2, or 3 and when n1 is 1, n1+n2+n3≥2, for example when n1 is 1, both n2 and n3 may not be 0.

The second organic material may be for example one of compounds of Group 1, but is not limited thereto.

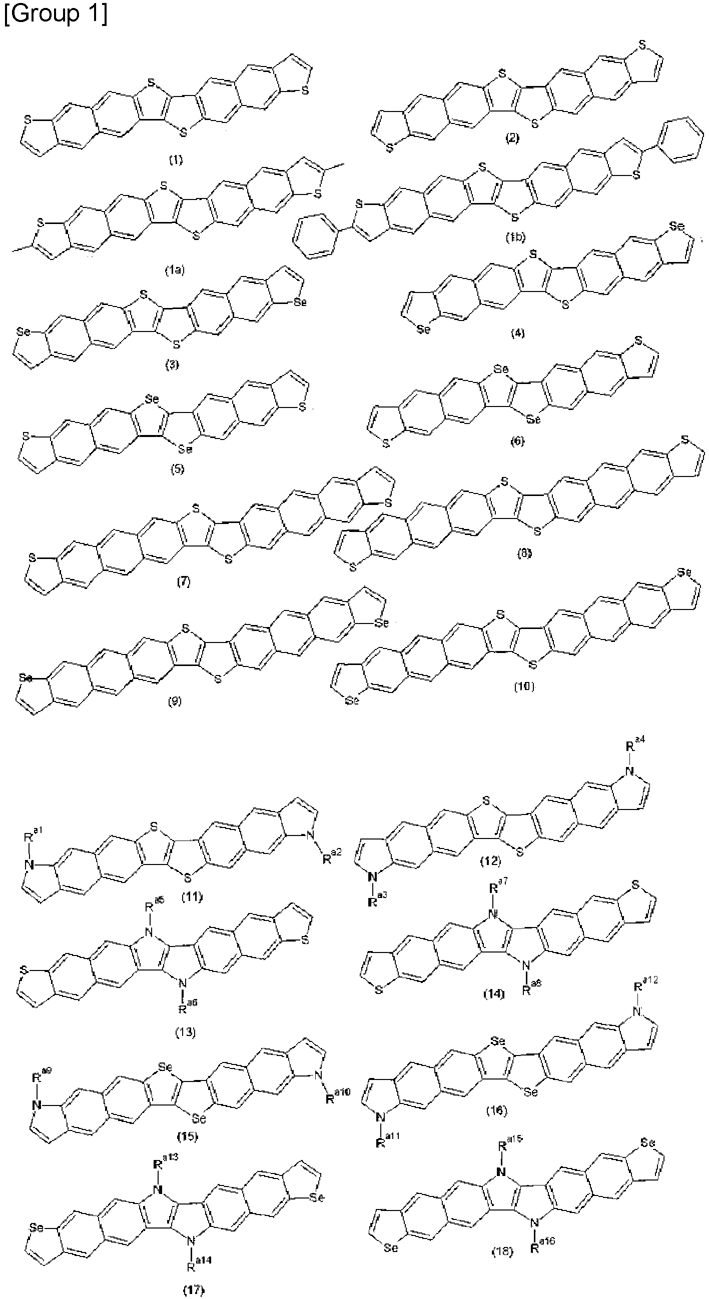

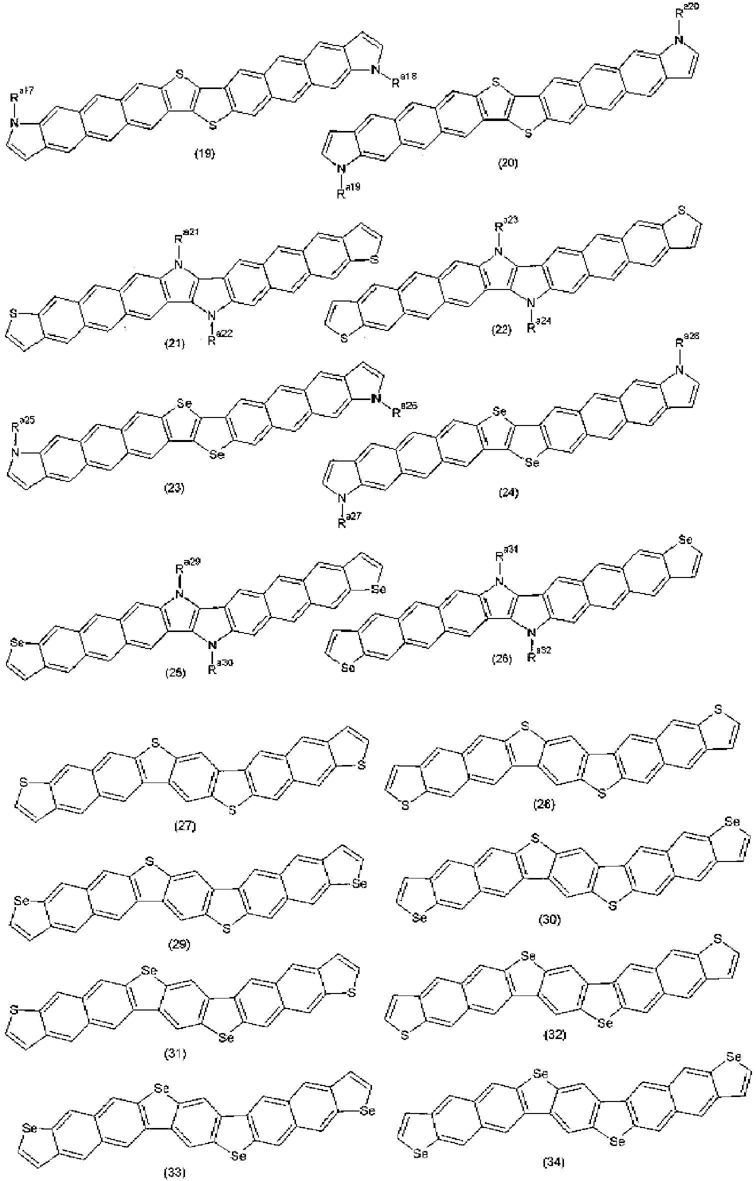

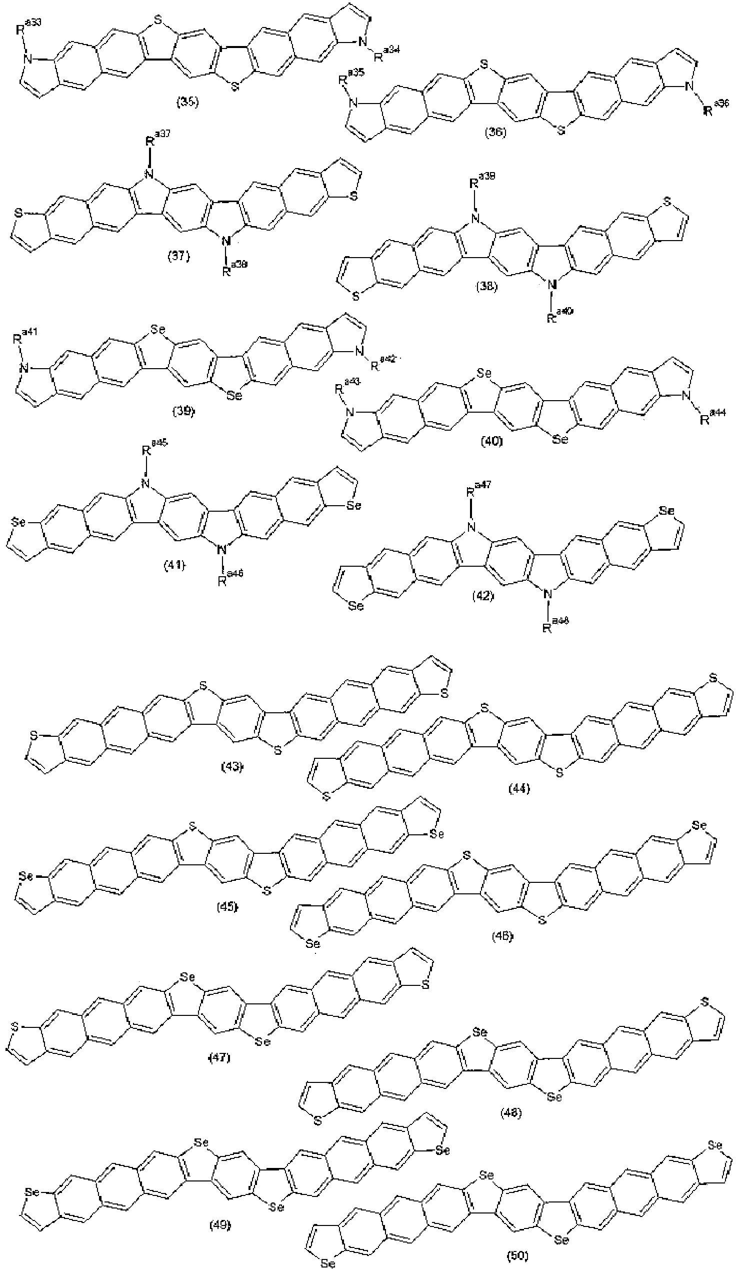

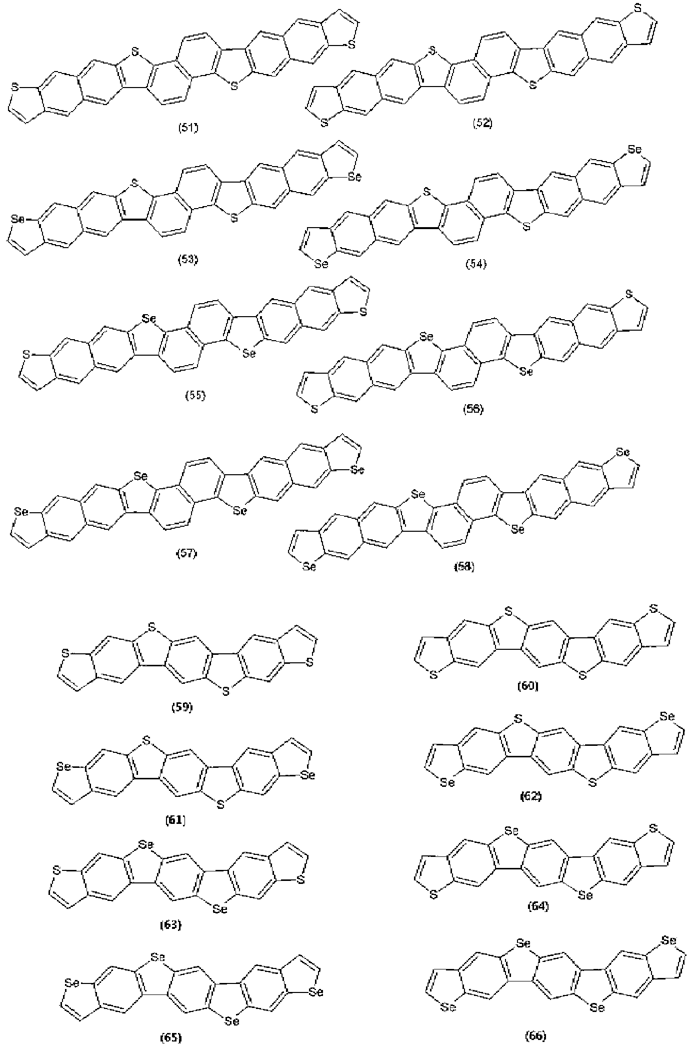

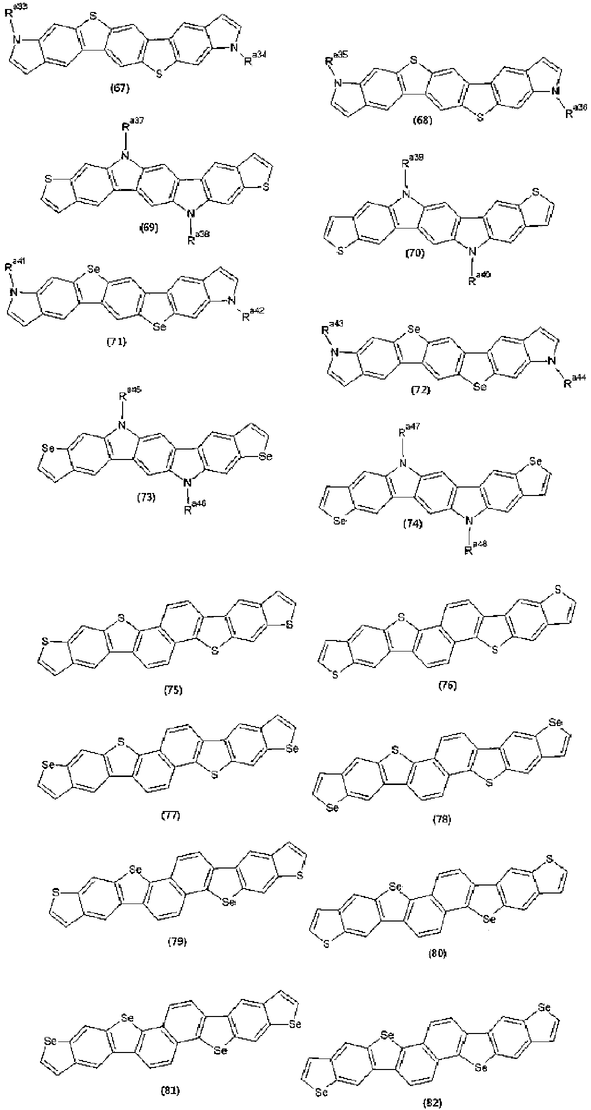

In the compounds of Group 1, hydrogen of each benzene, thiophene, sellenophene, and/or pyrrole may be may be replaced by a substituted or unsubstituted C1 to C30 alkyl group, a substituted or unsubstituted C1 to C30 alkoxy group, a substituted or unsubstituted C2 to C30 alkenyl group, a substituted or unsubstituted C2 to C30 alkynyl group, a substituted or unsubstituted C6 to C30 aryl group, a substituted or unsubstituted C2 to C30 heteroaryl group, a substituted or unsubstituted C7 to C30 arylalkyl group, a substituted or unsubstituted C2 to C30 heteroarylalkyl group, a substituted or unsubstituted C2 to C30 alkylheteroaryl group, a substituted or unsubstituted C5 to C30 cycloalkyl group, or a substituted or unsubstituted C2 to C30 heterocycloalkyl group.

In some example embodiments, the second organic material may be represented by Chemical Formula 3A or 3B, but is not limited thereto.

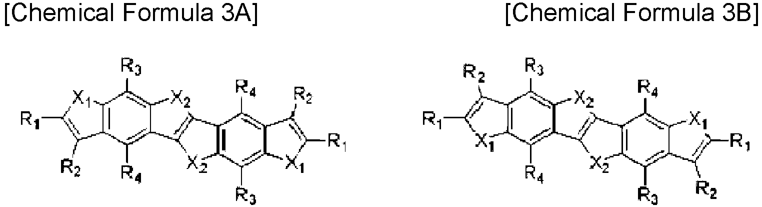

In Chemical Formulae 3A and 3B,
X¹ and X² may independently be O, S, Se, Te, or N-R^{a}, wherein R^{a} may be hydrogen, a substituted or unsubstituted C1 to C12 alkyl group, a substituted or unsubstituted C7 to C30 arylalkyl group, a substituted or unsubstituted C6 to C30 aryl group, a substituted or unsubstituted C1 to C12 alkoxy group, a substituted or unsubstituted C1 to C30 acyl group, a sulfonyl group, or a carbamate group, and
R¹ to R⁴ may independently be hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C1 to C30 heteroalkyl group, a substituted or unsubstituted C7 to C30 arylalkyl group, a substituted or unsubstituted C2 to C30 heteroarylalkyl group, a substituted or unsubstituted C5 to C20 cycloalkyl group, a substituted or unsubstituted C2 to C30 heterocycloalkyl group, a substituted or unsubstituted C6 to C30 aryl group, or a substituted or unsubstituted C2 to C30 heteroaryl group.

The organic semiconductor material represented by Chemical Formula 3A may be for example represented by Chemical Formula 3A-1 and the organic semiconductor material represented by Chemical Formula 3B may be for example represented by Chemical Formula 3B-1.

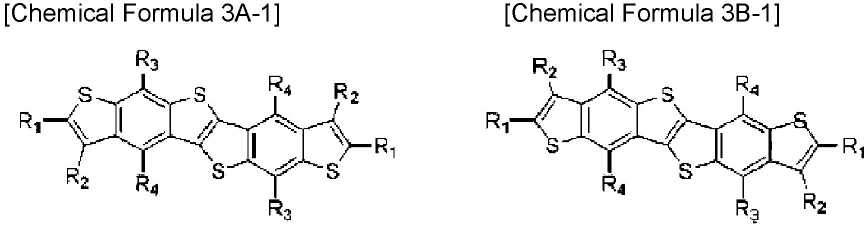

In Chemical Formulae 3A-1 and 3B-1, R¹ to R⁴ are the same as described above.

In some example embodiments, the second organic material may be for example one of compounds of Group 2, but is not limited thereto.

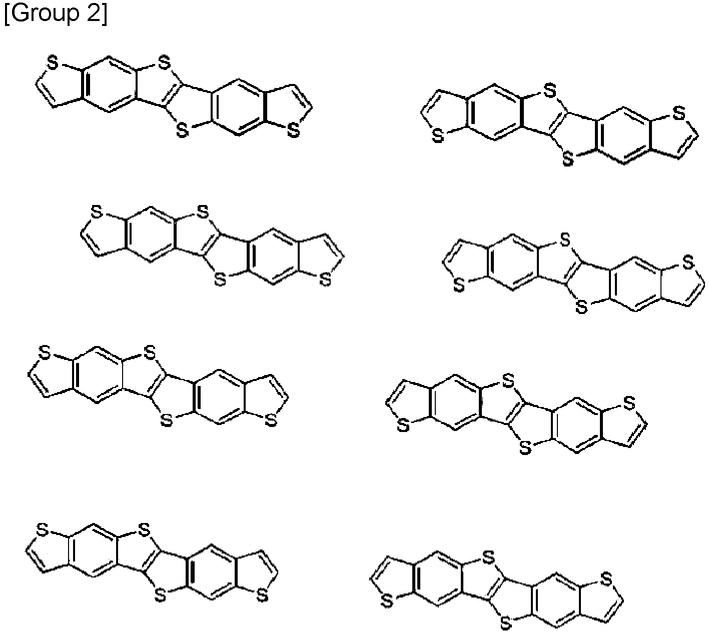

In the compounds of Group 2, hydrogen of each benzene and/or thiophene may be may be replaced by a substituted or unsubstituted C1 to C30 alkyl group, a substituted or unsubstituted C1 to C30 alkoxy group, a substituted or unsubstituted C2 to C30 alkenyl group, a substituted or unsubstituted C2 to C30 alkynyl group, a substituted or unsubstituted C6 to C30 aryl group, a substituted or unsubstituted C2 to C30 heteroaryl group, a substituted or unsubstituted C7 to C30 arylalkyl group, a substituted or unsubstituted C2 to C30 heteroarylalkyl group, a substituted or unsubstituted C2 to C30 alkylheteroaryl group, a substituted or unsubstituted C5 to C30 cycloalkyl group, or a substituted or unsubstituted C2 to C30 heterocycloalkyl group.

The organic auxiliary layer 40 further includes the semiconductor, which is the n-type semiconductor, in the photoelectric conversion layer 30. The second organic material and the n-type semiconductor are mixed. The second organic material and the n-type semiconductor may be mixed in a volume ratio of about 1:9 to about 9:1, for example about 2:8 to about 8:2, about 3:7 to about 7:3, about 4:6 to about 6:4, or about 5:5.

In some example embodiments, the organic auxiliary layer 40 may further include the p-type semiconductor and the n-type semiconductor in the photoelectric conversion layer 30. The second organic material and the p-type semiconductor/n-type semiconductor are mixed. The second organic material and the p-type semiconductor/the n-type semiconductor may be mixed in a volume ratio of about 1:9 to about 9:1, for example about 2:8 to about 8:2, about 3:7 to about 7:3, about 4:6 to about 6:4, or about 5:5.

In some example embodiments, the second organic material and the fullerene or fullerene derivative may be mixed. The second organic material and the fullerene or fullerene derivative may be mixed in a volume ratio of about 1:9 to about 9:1, for example about 2:8 to about 8:2, about 3:7 to about 7:3, about 4:6 to about 6:4, or about 5:5.

The organic auxiliary layer 40 may be thinner than the photoelectric conversion layer 30 and may have, for example a thickness of less than or equal to about 10 nm, or less than or equal to 5 nm. In some example embodiments, the organic auxiliary layer 40 may have a thickness of about 1 nm to about 10 nm, or about 1 nm to 5 nm.

As described above, the photoelectric conversion device 100 includes the organic auxiliary layer 40 between the first electrode 10 and the photoelectric conversion layer 30, so that extraction of charges (e.g., electrons) transferred from the photoelectric conversion layer 30 to the first electrode 10 may be effectively increased to reduce remaining charge carriers and to exhibit high charge extraction efficiency. Accordingly, it is possible to enhance the photoelectric conversion efficiency of the photoelectric conversion device 100 and to reduce an image lag which may be caused by remaining charge carriers, thereby effectively improving the after-image characteristics, thereby improving the performance of an organic sensor that includes the photoelectric conversion device 100.

The photoelectric conversion device 100 may further include an anti-reflection layer (not shown) on one surface of the first electrode 10 or the second electrode 20. The anti-reflection layer is disposed at a light incidence side and lowers reflectance of light of incident light and thereby light absorbance is further improved. In some example embodiments, when light is incident to the first electrode 10, the anti-reflection layer may be disposed on one surface of the first electrode 10, and when light is incident to the second electrode 20, anti-reflection layer may be disposed on one surface of the second electrode 20.

The anti-reflection layer may include, for example a material having a refractive index of about 1.6 to about 2.5, and may include for example at least one of metal oxide, metal sulfide, and an organic material having a refractive index within the ranges. The anti-reflection layer may include, for example a metal oxide such as aluminum-containing oxide, molybdenum-containing oxide, tungsten-containing oxide, vanadium-containing oxide, rhenium-containing oxide, niobium-containing oxide, tantalum-containing oxide, titanium-containing oxide, nickel-containing oxide, copper-containing oxide, cobalt-containing oxide, manganese-containing oxide, chromium-containing oxide, tellurium-containing oxide, or a combination thereof; metal sulfide such as zinc sulfide; or an organic material such as an amine derivative, but is not limited thereto.

In the photoelectric conversion device 100, when light enters from the first electrode 10 or the second electrode 20 and the photoelectric conversion layer 30 absorbs light in a particular (or, alternatively, predetermined) wavelength spectrum of light, excitons may be produced thereinside. The excitons are separated into holes and electrons in the photoelectric conversion layer 30, and the separated holes are transported to an anode that is one of the first electrode 10 and the second electrode 20 and the separated electrons are transported to the cathode that is the other of the first electrode 10 and the second electrode 20 so as to flow a current.

Hereinafter, a photoelectric conversion device according to some example embodiments is described.

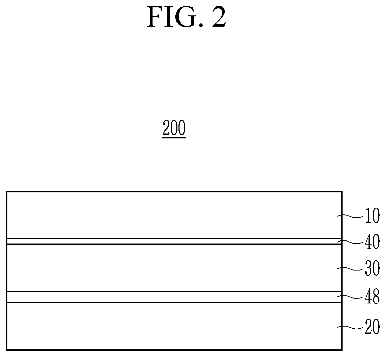

FIG. 2 is a cross-sectional view showing an example of a photoelectric conversion device according to some example embodiments.

Referring to FIG. 2, a photoelectric conversion device 200 according to some example embodiments includes a first electrode 10, a second electrode 20, a photoelectric conversion layer 30, and an organic auxiliary layer 40.

However, the photoelectric conversion device 200 according to some example embodiments includes a charge blocking layer 48 between the second electrode 20 and the photoelectric conversion layer 30. The charge blocking layer 48 may enhance photoelectric conversion efficiency by blocking charges (e.g., electrons) separated from the photoelectric conversion layer 30 from moving to the opposed electrode.

The charge blocking layer 48 may include, for example an organic material, an inorganic material, or an organic/inorganic material. The organic material may be an organic compound having hole or electron characteristics and the inorganic material may be, for example metal oxide such as molybdenum oxide, tungsten oxide, or nickel oxide.

The charge blocking layer 48 may include for example a visible light non-absorbing material that does not absorb light in a visible region substantially, for example a visible light non-absorbing organic material.

In some example embodiments, the visible light non-absorbing material may be a compound represented by Chemical Formula 4A or 4B, but is not limited thereto.

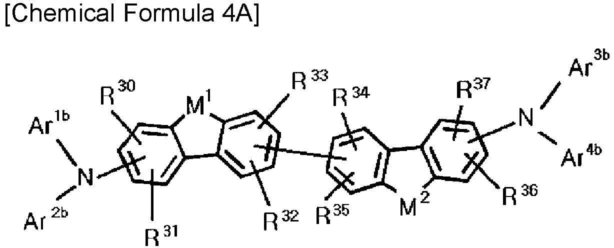

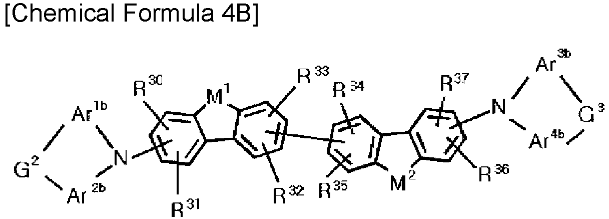

In Chemical Formula 4A or 4B,
M¹ and M² may independently be CRⁿR^{o}, SiR^{p}R^{q}, NR^{r}, O, S, Se, or Te,
Ar^{1b}, Ar^{2b}, Ar^{3b}, and Ar^{4b} may independently be a substituted or unsubstituted C6 to C30 aryl group or a substituted or unsubstituted C3 to C30 heteroaryl group,

G² and G³ may independently be a single bond, -(CR^{s}R^{t})ₙ₃ -, -O-, -S-, -Se-, - N=, -NR^{u}-, -SiR^{v}R^{w}-, or -GeR^{x}R^{y}-, wherein n3 is 1 or 2, and
R³⁰ to R³⁷ and Rⁿ to R^{y} may independently be hydrogen, a substituted or unsubstituted C1 to C30 alkyl group, a substituted or unsubstituted C6 to C30 aryl group, a substituted or unsubstituted C3 to C30 heterocyclic group, a substituted or unsubstituted C1 to C6 alkoxy group, a halogen, or a cyano group.

In some example embodiments, the visible light non-absorbing material may be a compound represented by Chemical Formula 4A-1 or 4B-1, but is not limited thereto.

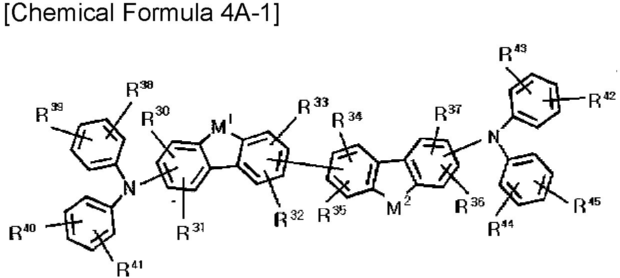

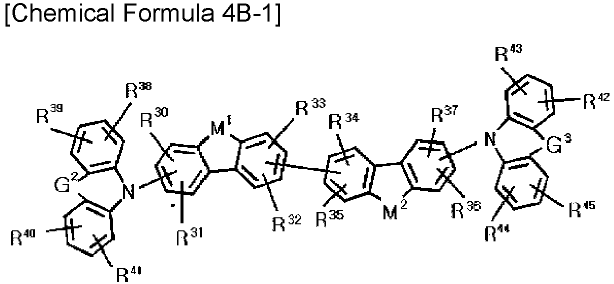

In Chemical Formula 4A-1 or 4B-1,
M¹, M², G², G³, and R³⁰ to R³⁷ are the same as described above, and
R³⁸ to R⁴⁵ may independently be hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C1 to C6 alkoxy group, a halogen, or a cyano group.

In some example embodiments, the visible light non-absorbing material may be a compound represented by Chemical Formula 4A-1a or 4B-1a, but is not limited thereto.

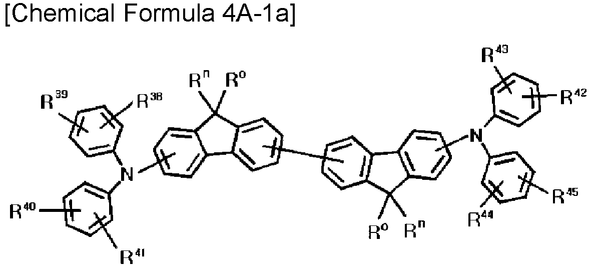

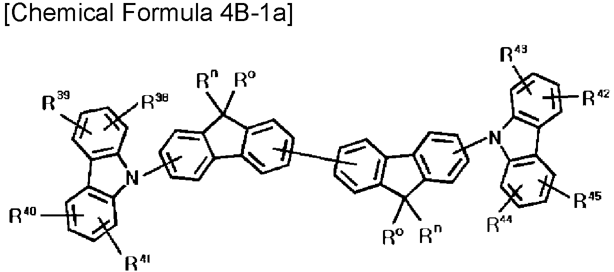

In Chemical Formula 4A-1a or 4B-1a, R³⁸ to R⁴⁵ and R^{o} and Rⁿ are the same as described above.

The photoelectric conversion device 200 according to some example embodiments further includes the charge blocking layer 48 between the second electrode 20 and the photoelectric conversion layer 30 in addition to the organic auxiliary layer 40 between the first electrode 10 and the photoelectric conversion layer 30, and thereby holes and electrons separated from the photoelectric conversion layer 30 may be transferred to each anode and cathode efficiently to further increase charge extraction efficiency.

Hereinafter, a photoelectric conversion device according to some example embodiments is described.

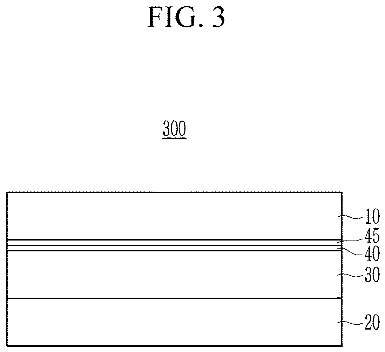

FIG. 3 is a cross-sectional view showing an example of a photoelectric conversion device according to some example embodiments.

Referring to FIG. 3, a photoelectric conversion device 300 according to some example embodiments includes a first electrode 10, a second electrode 20, a photoelectric conversion layer 30, and an organic auxiliary layer 40.

In some example embodiments, including the example embodiments shown in FIG. 3, the photoelectric conversion device 300 further includes an inorganic nanolayer 45.

The inorganic nanolayer 45 may be disposed between the first electrode 10 and the photoelectric conversion layer 30, and may be for example in contact with the first electrode 10. In some example embodiments, the organic auxiliary layer 40 may be in contact with the photoelectric conversion layer 30, the inorganic nanolayer 45 may be in contact with the first electrode 10, and the organic auxiliary layer 40 and the inorganic nanolayer 45 may be in contact with each other.

The inorganic nanolayer 45 may be a very thin film of a several nanometers in thickness and may have a thickness of, in some example embodiments, less than or equal to 5 nm, less than or equal to about 3 nm, or less than or equal to about 2 nm. The inorganic nanolayer 45 may have, for example a thickness of about 1 nm to 5 nm, about 1 nm to about 3 nm, or about 1 nm to about 2 nm.

The inorganic nanolayer 45 may include an inorganic material having a lower work function than the first electrode 10. In some example embodiments, a work function of the inorganic nanolayer 45 may be less than a work function of the first electrode 10 by greater than or equal to about 0.5 eV. In some example embodiments, the work function of the first electrode 10 may be greater than or equal to about 4.5 eV and the work function of the inorganic nanolayer 45 may be less than or equal to about 4.0 eV. In some example embodiments, the work function of the first electrode 10 may be greater than or equal to about 4.5 eV and the work function of the inorganic nanolayer 45 may be less than or equal to about 3.5 eV. In some example embodiments, the work function of the first electrode 10 may be greater than or equal to about 4.5 eV and the work function of the inorganic nanolayer 45 may be less than or equal to about 3.0 eV. In some example embodiments, the work function of the first electrode 10 may be greater than or equal to about 4.5 eV and the work function of the inorganic nanolayer 45 may be less than or equal to about 2.8 eV. In some example embodiments, the work function of the first electrode 10 may be about 4.5 eV to about 5.0 eV and the work function of the inorganic nanolayer 45 may be about 1.5 eV to about 4.0 eV, about 1.5 eV to about 3.5 eV, about 1.5 eV to about 3.0 eV, or about 1.5 eV to about 2.8 eV.

The inorganic nanolayer 45 may include, in some example embodiments, a lanthanide element, calcium (Ca), potassium (K), aluminum (Al), or an alloy thereof. The lanthanide element may include, for example ytterbium (Yb).

As described above, the inorganic nanolayer 45 may be in contact with the surface of the first electrode 10 between the first electrode 10 and the photoelectric conversion layer 30, and it may have a thin thickness compared with the first electrode 10. Thus, the inorganic nanolayer 45 may function as a surface-treatment layer of the first electrode 10 on the surface of the first electrode 10.

In some example embodiments, the effective work function on ("at") the surface of the first electrode 10 may be different from the work function of the conductor (e.g., the transparent conductor or the reflective conductor) of the first electrode 10 by the influences of the inorganic nanolayer 45. In some example embodiments, the effective work function on the surface of the first electrode 10 may be less than the work function of the conductor (e.g., the transparent conductor or the reflective conductor) of the first electrode 10. In some example embodiments, the effective work function on the surface of the first electrode 10 may be the same as the work function of the inorganic nanolayer 45 or may have a medium value between the work function of the inorganic nanolayer 45 and the work function of the first electrode 10.

In some example embodiments, the work functions of the conductor (e.g., transparent conductor or reflective conductor) for the first electrode 10 may be greater than or equal to about 4.5 eV, and the effective work function on ("at") the surface of the first electrode 10 may be less than or equal to about 4.0 eV. In some example embodiments, the work function of the conductor (e.g., transparent conductor or reflective conductor) for the first electrode 10 may be greater than or equal to about 4.5 eV, and the effective work function on ("at") the surface of the first electrode 10 may be less than or equal to about 3.5 eV. In some example embodiments, the work function of the conductor (e.g., transparent conductor or reflective conductor) for the first electrode 10 may be greater than or equal to about 4.5 eV, and the effective work function on the surface of the first electrode 10 may be less than or equal to about 3.0 eV. In some example embodiments, the work function of the conductor (e.g., transparent conductor or reflective conductor) for the first electrode 10 may be greater than or equal to about 4.5 eV, and the effective work function on the surface of the first electrode 10 may be less than or equal to about 2.8 eV. In some example embodiments, the work function of the conductor (e.g., transparent conductor or reflective conductor) for the first electrode 10 may be about 4.5 eV to about 5.0 eV, and the effective work function on ("at") the surface of the first electrode 10 may be about 1.5 eV to about 4.0 eV, about 1.5 eV to about 3.5 eV, about 1.5 eV to about 3.0 eV, or about 1.5 eV to about 2.8 eV.

By lowering the work function on ("at") the surface of the first electrode 10 as described above, extraction of charges (e.g., electrons) passing the organic auxiliary layer 40 from the photoelectric conversion layer 30 and moving to the first electrode 10 may be further facilitated, and remaining charges may be further reduced to show a higher charge extraction efficiency.

Hereinafter, a photoelectric conversion device according to some example embodiments is described.

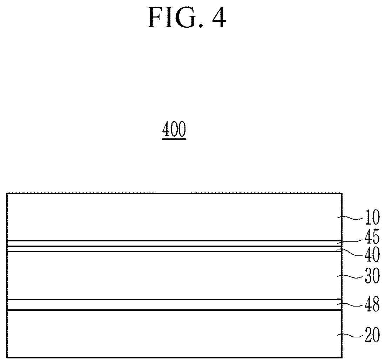

FIG. 4 is a cross-sectional view showing an example of a photoelectric conversion device according to some example embodiments.

Referring to FIG. 4, a photoelectric conversion device 400 according to some example embodiments includes a first electrode 10, a second electrode 20, a photoelectric conversion layer 30, an organic auxiliary layer 40, and an inorganic nanolayer 45.

However, the photoelectric conversion device 400 according to some example embodiments further includes a charge blocking layer 48 between the second electrode 20 and the photoelectric conversion layer 30. The charge blocking layer 48 may enhance photoelectric conversion efficiency by blocking charges (e.g., electrons) separated from the photoelectric conversion layer 30 from moving to the opposed electrode and details thereof are the same as described above.

The photoelectric conversion devices 100, 200, 300, and 400 may be applied to various electronic devices, for example a solar cell, an organic sensor, a photodetector, and a photosensor, but is not limited thereto.

The photoelectric conversion devices 100, 200, 300, and 400 may be for example applied to an organic sensor.

Hereinafter, examples of organic sensors using the photoelectric conversion devices 100, 200, 300, and 400 will be described with reference to the drawings.

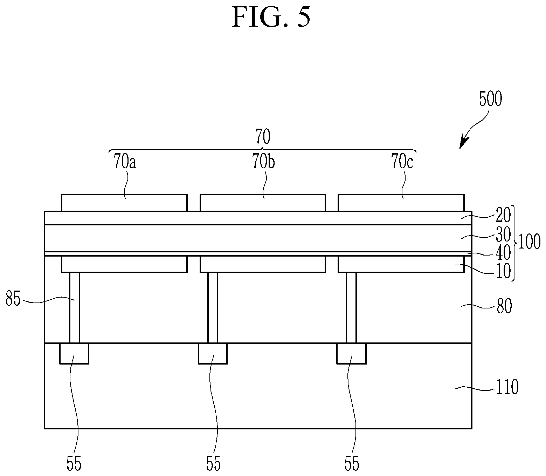

FIG. 5 is a schematic cross-sectional view showing an example of an organic sensor according to some example embodiments.

In some example embodiments, the organic sensor may be an organic CMOS organic sensor.

Referring to FIG. 5, an organic sensor 500 according to some example embodiments includes a semiconductor substrate 110, an insulation layer 80, a photoelectric conversion device 100, and a color filter layer 70.

The semiconductor substrate 110 may be a silicon substrate, and is integrated with the transmission transistor (not shown) and the charge storage 55. The transmission transistor and/or the charge storage 55 may be integrated in each pixel.

A metal wire (not shown) and a pad (not shown) are formed on the semiconductor substrate 110. In order to decrease signal delay, the metal wire and pad may be made of a metal having low resistivity, in some example embodiments, aluminum (Al), copper (Cu), silver (Ag), and alloys thereof, but are not limited thereto.

The insulation layer 80 is formed on the metal wire and the pad. The insulation layer 80 may be made of an inorganic insulating material such as a silicon oxide and/or a silicon nitride, or a low dielectric constant (low K) material such as SiC, SiCOH, SiCO, and SiOF. The insulation layer 80 has a trench 85 exposing the charge storage 55. The trench 85 may be filled with fillers.

The photoelectric conversion device 100 is formed on the insulation layer 80. The photoelectric conversion device 100 includes a first electrode 10, an organic auxiliary layer 40, a photoelectric conversion layer 30, and a second electrode 20 as described above. Details thereof are the same as described above.

A color filter layer 70 is formed on the photoelectric conversion device 100. The color filter layer 70 includes a blue filter 70a formed in a blue pixel, a red filter 70b formed in a red pixel, and a green filter 70c formed in a green pixel. However, the color filter layer 70 may include a cyan filter, a magenta filter, and/or a yellow filter instead of the above color filters or may further include them in addition to the above color filters. It will be understood that a color filter is configured to selectively transmit a particular wavelength spectrum of light. Where a color filter overlaps a photo-sensing device, the color filter may be configured to selectively transmit a particular wavelength spectrum of light to a photo-sensing device so that the photo-sensing device is configured to absorb, and convert into electrical signals, the particular wavelength spectrum of light.

Focusing lens (not shown) may be further formed on the color filter layer 70. The focusing lens may control a direction of incident light and gather the light in one region. The focusing lens may have a shape of, in some example embodiments, a cylinder or a hemisphere, but is not limited thereto.

Even though the structure including the stacked photoelectric conversion device 100 of FIG. 1 is for example illustrated in FIG. 5, structures in which the photoelectric conversion devices 200, 300, and 400 of FIGS. 2, 3, and 4 are stacked may be applied in the same manner.

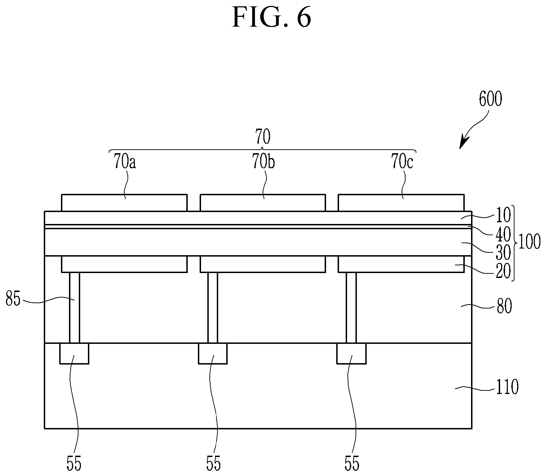

FIG. 6 is a schematic cross-sectional view showing an example of an organic sensor according to some example embodiments.

Referring to FIG. 6, an organic sensor 600 according to some example embodiments includes a semiconductor substrate 110 integrated with a transmission transistor (not shown) and a charge storage 55, an insulation layer 80, a photoelectric conversion device 100, and a color filter layer 70.

However, in the organic sensor 600 according to some example embodiments, the positions of the first electrode 10 and the second electrode 20 of the photoelectric conversion device 100 are changed. That is, the first electrode 10 may be a light-receiving electrode.

Even though the structure including the stacked photoelectric conversion device 100 of FIG. 1 is for example illustrated in FIG. 6, structures in which the photoelectric conversion devices 200, 300, and 400 of FIGS. 2, 3, and 4 are stacked may be applied in the same manner.

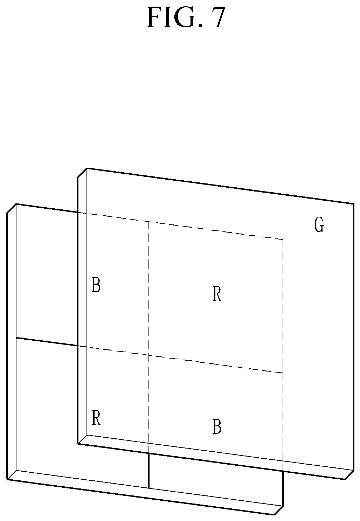

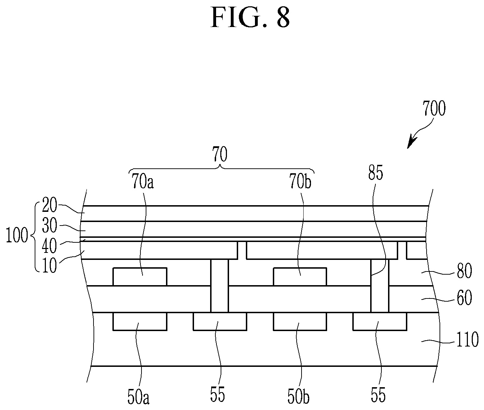

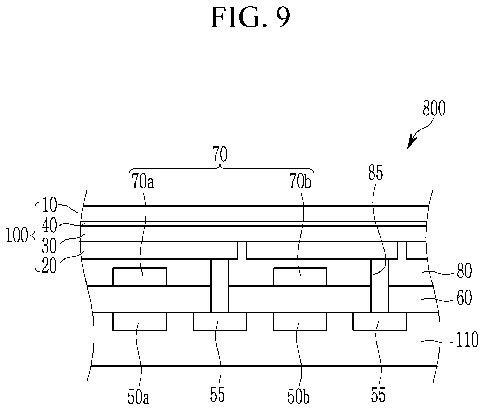

FIG. 7 is a schematic top plan view showing an example of an organic sensor according to some example embodiments and FIGS. 8 and 9 are schematic cross-sectional view showing examples of the organic sensor of FIG. 7, respectively.

Referring to FIGS. 7 and 8, an organic sensor 700 according to some example embodiments includes a semiconductor substrate 110 integrated with photo-sensing devices 50a and 50b, a transmission transistor (not shown), and a charge storage 55, a lower insulation layer 60, a color filter layer 70, an upper insulation layer 80, and a photoelectric conversion device 100.

The semiconductor substrate 110 may be a silicon substrate, and is integrated with the photo-sensing devices 50a and 50b, the transmission transistor (not shown), and the charge storage 55. The photo-sensing devices 50a and 50b may be photodiodes.

The photo-sensing devices 50a and 50b, the transmission transistor, and/or the charge storage 55 may be integrated in each pixel, and as shown in the drawing, the photo-sensing devices 50a and 50b may be respectively included in a blue pixel and a red pixel and the charge storage 55 may be included in a green pixel.

The photo-sensing devices 50a and 50b sense light, the information sensed by the photo-sensing devices may be transferred by the transmission transistor, the charge storage 55 is electrically connected to the photoelectric conversion device 100 that will be described later, and the information of the charge storage 55 may be transferred by the transmission transistor.

A metal wire (not shown) and a pad (not shown) are formed on the semiconductor substrate 110. In order to decrease signal delay, the metal wire and pad may be made of a metal having low resistivity, in some example embodiments, aluminum (Al), copper (Cu), silver (Ag), and alloys thereof, but are not limited thereto. Further, it is not limited to the structure, and the metal wire and pad may be disposed under the photo-sensing devices 50a and 50b.

The lower insulation layer 60 is formed on the metal wire and the pad. The lower insulation layer 60 may be made of an inorganic insulating material such as a silicon oxide and/or a silicon nitride, or a low dielectric constant (low K) material such as SiC, SiCOH, SiCO, and SiOF. The lower insulation layer 60 has a trench 85 exposing the charge storage 55. The trench 85 may be filled with fillers.

A color filter layer 70 is formed on the lower insulation layer 60. The color filter layer 70 includes a blue filter 70a formed in a blue pixel and a red filter 70b in a red pixel. In some example embodiments, a green filter is not included, but a green filter may be further included. For another example, the color filter layer 70 may be disposed on the photoelectric conversion device 100.

The upper insulation layer 80 is formed on the color filter layer 70. The upper insulation layer 80 may eliminate a step caused by the color filter layer 70 and smoothen the surface. The upper insulation layer 80 and the lower insulation layer 60 may include a contact hole (not shown) exposing a pad, and a trench 85 trench 85.

The photoelectric conversion device 100 is formed on the upper insulation layer 80. The photoelectric conversion device 100 includes the first electrode 10, the organic auxiliary layer 40, the photoelectric conversion layer 30, and the second electrode 20 as described above. Details are the same as described above.

Focusing lens (not shown) may be further formed on the photoelectric conversion device 100. The focusing lens may control a direction of incident light and gather the light in one region. The focusing lens may have a shape of, in some example embodiments, a cylinder or a hemisphere, but is not limited thereto.

Referring to FIG. 9, the organic sensor 800 according to some example embodiments includes a semiconductor substrate 110 integrated with photo-sensing devices 50a and 50b, a transmission transistor (not shown), and a charge storage 55, a lower insulation layer 60, a color filter layer 70, an upper insulation layer 80, and a photoelectric conversion device 100.

However, in the organic sensor 800 according to some example embodiments, the positions of the first electrode 10 and the second electrode 20 are changed. That is, the first electrode 10 may be a light-receiving electrode.

Even though the structure including the stacked photoelectric conversion device 100 of FIG. 1 is for example illustrated in FIGS. 8 and 9, structures in which the photoelectric conversion devices 200, 300, and 400 of FIGS. 2, 3, and 4 are stacked may be applied in the same manner.

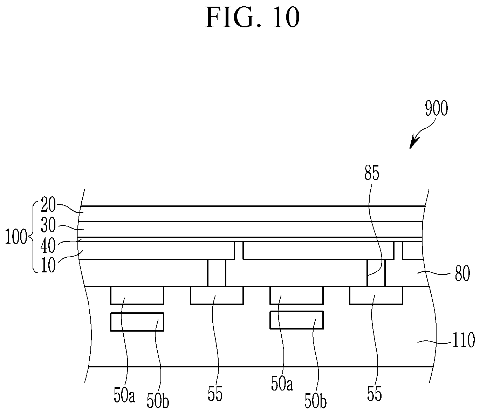

FIG. 10 is a schematic cross-sectional view showing an example of an organic sensor according to some example embodiments.

An organic sensor 900 according to some example embodiments includes a semiconductor substrate 110 integrated with photo-sensing devices 50a and 50b, a transmission transistor (not shown), and a charge storage 55, an insulation layer 80 having a trench 85, and a photoelectric conversion device 100.

However, in the organic sensor 900 according to some example embodiments, the photo-sensing devices 50a and 50b are stacked in a vertical direction and the color filter layer 70 is omitted. The photo-sensing devices 50a and 50b are electrically connected to charge storage (not shown) and may be transferred by the transmission transistor. The photo-sensing devices 50a and 50b may selectively absorb light in each wavelength spectrum of light depending on a stacking depth.

Even though the structure including the stacked photoelectric conversion device 100 of FIG. 1 is for example illustrated in FIG. 10, structures in which the photoelectric conversion devices 200, 300, and 400 of FIGS. 2, 3, and 4 are stacked may be applied in the same manner.

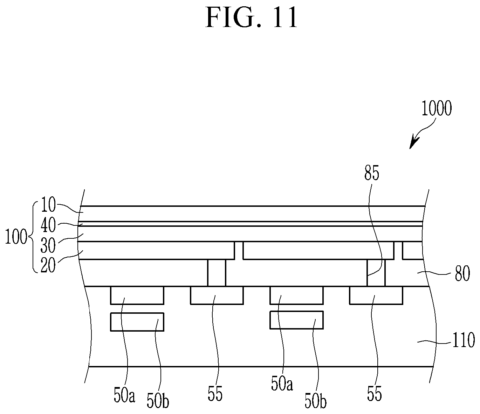

FIG. 11 is a schematic cross-sectional view showing an example of an organic sensor according to some example embodiments.

Referring to FIG. 11, an organic sensor 1000 according some example embodiments includes a semiconductor substrate 110 integrated with photo-sensing devices 50a and 50b, a transmission transistor (not shown), and a charge storage 55, an insulation layer 80 having a trench 85, and a photoelectric conversion device 100. However, in the organic sensor 1000 according to some example embodiments, the positions of the first electrode 10 and the second electrode 20 are changed. That is, the first electrode 10 may be a light-receiving electrode.

Even though the structure including the stacked photoelectric conversion device 100 of FIG. 1 is for example illustrated in FIG. 11, structures in which the photoelectric conversion devices 200, 300, and 400 of FIGS. 2, 3, and 4 are stacked may be applied in the same manner.

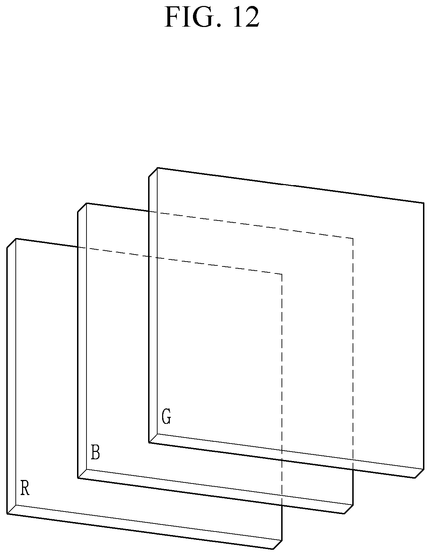

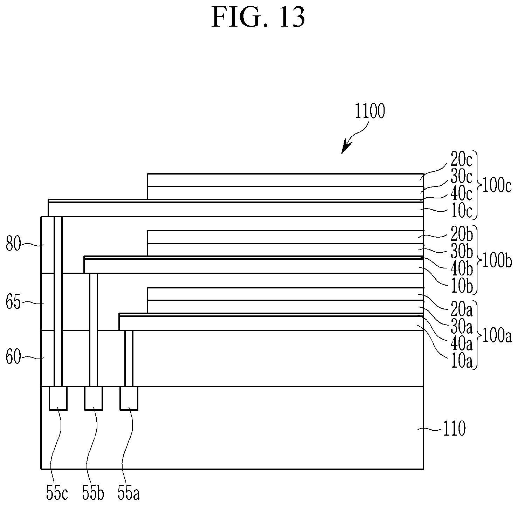

FIG. 12 is a schematic top plan view showing an example of an organic sensor according to some example embodiments, and FIG. 13 is a schematic cross-sectional view showing an example of the organic sensor of FIG. 12.

An organic sensor 1100 according to some example embodiments has a structure in which a green photoelectric conversion device selectively absorbing light in a green wavelength spectrum of light, a blue photoelectric conversion device selectively absorbing light in a blue wavelength spectrum of light, and a red photoelectric conversion device selectively absorbing light in a red wavelength spectrum of light are stacked.

The organic sensor 1100 according to some example embodiments includes a semiconductor substrate 110, a lower insulation layer 60, an intermediate insulation layer 65, an upper insulation layer 80, a first photoelectric conversion device 100a, a second photoelectric conversion device 100b, and a third photoelectric conversion device 100c.

The semiconductor substrate 110 may be a silicon substrate, and is integrated with the transmission transistor (not shown) and the charge storage 55a, 55b, and 55c. A metal wire (not shown) and a pad (not shown) are formed on the semiconductor substrate 110, and the lower insulation layer 60 is formed on the metal wire and the pad.

The first photoelectric conversion device 100a is formed on the lower insulation layer 60.

The first photoelectric conversion device 100a includes a first electrode 10a and a second electrode 20a facing each other, and a photoelectric conversion layer 30a and an organic auxiliary layer 40a disposed between the first electrode 10a and the second electrode 20a. The first electrode 10a, the second electrode 20a, the photoelectric conversion layer 30, and the organic auxiliary layer 40a are the same as described above, and the photoelectric conversion layer 30a may selectively absorb light in one of red, blue, and green wavelength spectra of light. In some example embodiments, the first photoelectric conversion device 100a may be a red photoelectric conversion device.

The intermediate insulation layer 65 may be formed on the first photoelectric conversion device 100a.

The second photoelectric conversion device 100b may be formed on the intermediate insulation layer 65.

The second photoelectric conversion device 100b includes a first electrode 10b and a second electrode 20b, and a photoelectric conversion layer 30b and an organic auxiliary layer 40b between the first electrode 10b and the second electrode 20b. The first electrode 10b, the second electrode 20b, the photoelectric conversion layer 30b, and the organic auxiliary layer 40b are the same as described above, and the photoelectric conversion layer 30b may selectively absorb light in one of red, blue, and green wavelength spectra of light. In some example embodiments, the first photoelectric conversion device 100b may be a blue photoelectric conversion device.

The upper insulation layer 80 may be formed on the second photoelectric conversion device 100b. The lower insulation layer 60, the intermediate insulation layer 65, and the upper insulation layer 80 have a plurality of trenches 85 exposing the charge storages 55a, 55b, and 55c.

The third photoelectric conversion device 100c is formed on the upper insulation layer 80. The third photoelectric conversion device 100c includes a first electrode 10c and a second electrode 20c facing each other, and a photoelectric conversion layer 30c and an organic auxiliary layer 40c disposed between the first electrode 10c and the second electrode 20c. The first electrode 10c, the second electrode 20c, the photoelectric conversion layer 30c, and the organic auxiliary layer 40c are the same as described above, and the photoelectric conversion layer 30c may selectively absorb light in one of red, blue, and green wavelength spectra of light. In some example embodiments, the third photoelectric conversion device 100c may be a green photoelectric conversion device and may be the photoelectric conversion device 100.

Focusing lens (not shown) may be further formed on the photoelectric conversion device 100c. The focusing lens may control a direction of incident light and gather the light in one region. The focusing lens may have a shape of, in some example embodiments, a cylinder or a hemisphere, but is not limited thereto.

In the drawing, even though as the first photoelectric conversion device 100a, the second photoelectric conversion device 100b, and the third photoelectric conversion device 100c, the photoelectric conversion device 100 of FIG. 1 is for example illustrated, photoelectric conversion devices 200, 300, and 400 of FIG. 2, 3, or 4 may be applied in the same manner.

In the drawing, the first photoelectric conversion device 100a, the second photoelectric conversion device 100b, and the third photoelectric conversion device 100c are sequentially stacked, but the present disclosure is not limited thereto, and they may be stacked in various orders.

As described above, the first photoelectric conversion device 100a, the second photoelectric conversion device 100b, and the third photoelectric conversion device 100c are stacked, and thus the size of an organic sensor may be reduced to realize a down-sized organic sensor.

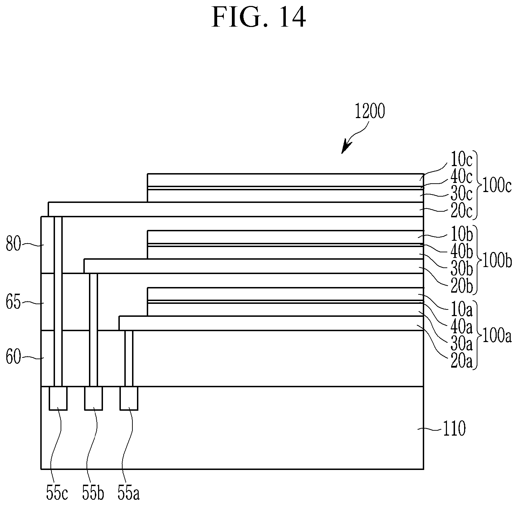

FIG. 14 is a schematic cross-sectional view showing an example of an organic sensor according to some example embodiments.

Referring to FIG. 14, an organic sensor 1200 according some example embodiments includes a semiconductor substrate 110, a lower insulation layer 60, an intermediate insulation layer 65, an upper insulation layer 80, a first photoelectric conversion device 100a, a second photoelectric conversion device 100b, and a third photoelectric conversion device 100c. However, the positions of the first electrode 10 and the second electrode 20 of the first photoelectric conversion device 100a, the second photoelectric conversion device 100b, and the third photoelectric conversion device 100c are changed. That is, the first electrode 10 may be a light-receiving electrode.

The photoelectric conversion device and the organic sensor may be applied to various electronic devices, for example a mobile phone or a digital camera, but are not limited thereto.

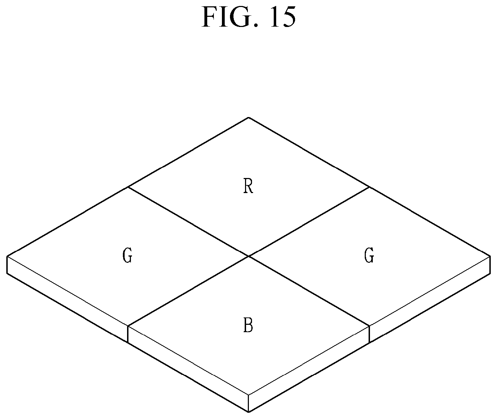

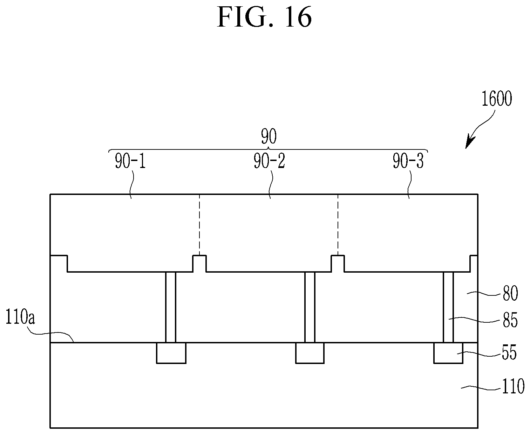

FIG. 15 is a schematic top plan view showing an example of an organic sensor according to some example embodiments, and FIG. 16 is a schematic cross-sectional view showing an example of the organic sensor of FIG. 15.

As shown with reference to FIGS. 15-16, an organic sensor 1600 may include a photoelectric conversion device 90 that itself includes a plurality of photoelectric conversion devices 90-1, 90-2, and 90-3 on a semiconductor substrate 110, where the plurality of photoelectric conversion devices 90-1, 90-2, and 90-3 are configured to convert different wavelength spectra of light (e.g., different ones of blue light, green light, or red light) into electric signals, respectively. As shown in FIG. 16, the separate photoelectric conversion devices 90-1 to 90-3 may be horizontally arranged on the semiconductor substrate 110 such that the photoelectric conversion devices 90-1 to 90-3 are partially or entirely overlapped with each other in a direction that extends in parallel with a top surface 110a of the semiconductor substrate 110. As shown each separate photoelectric conversion device 90-1 to 90-3 is connected to a separate charge storage 55 that is integrated into the semiconductor substrate 110 via a separate trench 85.

Each photoelectric conversion device 90-1 to 90-3 of the photoelectric conversion device 90 may be any one of the photoelectric conversion devices 100-400 described herein. In some example embodiments, separate photoelectric conversion devices 90-1 to 90-3 may include different portions of a common, continuous layer that extends continuously between two or more of the photoelectric conversion devices 90-1 to 90-3. In some example embodiments, the photoelectric conversion devices 90-1 to 90-3 may share a common first electrode 10 and a common second electrode 20. In another example, two or more of the photoelectric conversion devices 90-1 to 90-3 may have different photoelectric conversion layers 30 that are configured to absorb different wavelength spectra of incident light. In another example, two or more of the photoelectric conversion devices 90-1 to 90-3 may have different configurations of organic auxiliary layers 40, different inorganic nanolayers 45, different charge blocking layers 48, some combination thereof, or the like, such that, for example one or more of the photoelectric conversion devices 90-1 to 90-3 may include a charge blocking layer 48 and another one or more of the photoelectric conversion devices 90-1 to 90-3 may omit a charge blocking layer 48. Other structures of organic sensor 1600 may be are the same as one or more of the organic sensors described with reference to any of FIGS. 5-14.

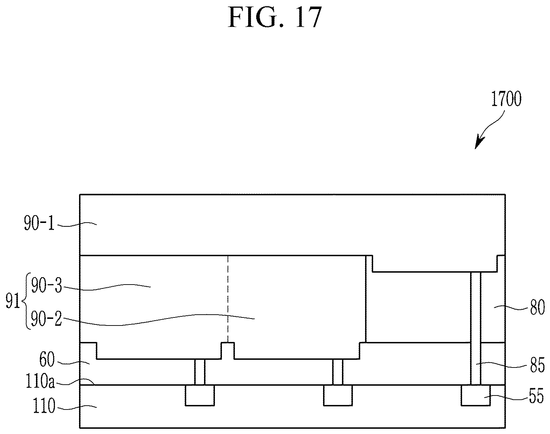

FIG. 17 is a schematic cross-sectional view of an organic sensor according to some example embodiments.

Referring to FIG. 17, an organic sensor 1700 may include a semiconductor substrate 110 and photoelectric conversion devices 90-1 and 91 that are stacked on each other so as to at least partially overlap in a direction extending perpendicular to the top surface 110a of the semiconductor substrate 110, and wherein at least one of the photoelectric conversion devices 90-1 and 91 further includes multiple photoelectric conversion devices 90-2 and 90-3 that are arranged so as to overlap in a direction extending parallel to the top surface 110a of the semiconductor substrate 110, and where the plurality of photoelectric conversion devices 90-1, 90-2, and 90-3 are configured to convert different wavelength spectra of light (e.g., different ones of blue light, green light, or red light) into electric signals, respectively. It will be understood that, in some example embodiments, photoelectric conversion device 90-1 includes multiple, horizontally-arranged photoelectric conversion devices configured to absorb different wavelengths spectra of light while photoelectric conversion device 91 is limited to a single photoelectric conversion device that is configured to absorb a single wavelength spectrum of light. In some example embodiments, including the example embodiments shown in FIG. 17, an entirety of the photoelectric conversion device 91 overlaps a limited portion of the photoelectric conversion device 90-1 in the direction extending perpendicular to the top surface 110a and a remainder portion of the photoelectric conversion device 90-1 that is exposed by the photoelectric conversion device 91 is covered by insulation layer 80. However, it will be understood that in some example embodiments an entirety of the photoelectric conversion device 90-1 overlaps a limited portion of the photoelectric conversion device 91 in the direction extending perpendicular to the top surface 110a. Other structures of organic sensor 1700 may be are the same as one or more of the organic sensors described with reference to any of FIGS. 5-14.

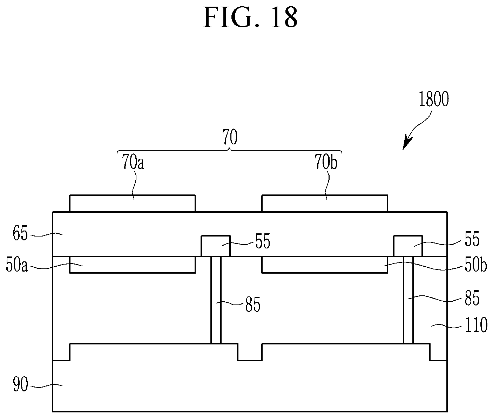

FIG. 18 is a schematic cross-sectional view of an organic sensor according to some example embodiments.

Referring to FIG. 18, an organic sensor 1800 according to some example embodiments includes a semiconductor substrate 110 integrated with photo-sensing devices 50a and 50b, a transmission transistor (not shown) and a charge storage 55, an intermediate insulation layer 65, and a color filter layer 70 on the semiconductor substrate 110, and a photoelectric conversion device 90 under the semiconductor substrate 110. The photoelectric conversion device 90 shown in FIG. 18 may be any of the example embodiments of photoelectric conversion devices described herein. As shown in FIG. 18, the photoelectric conversion device 90 may be on (e.g., above or beneath) the semiconductor substrate 110, such that the color filter layer 70 is distal from the photoelectric conversion device 90 in relation to the photo-sensing devices 50a and 50b. Other structures of organic sensor 1800 may be are the same as one or more of the organic sensors described with reference to any of FIGS. 5-14.

It will be understood that, where an organic sensor includes a photo-sensing device and a photoelectric conversion device, the photo-sensing device and the photoelectric conversion device may be configured to absorb different, first and second wavelength spectra of light and convert said absorbed light into electric signals.

While FIG. 18 illustrates example embodiments where color filters 70a, 70b of a color filter layer overlap separate, respective photo-sensing devices 50a, 50b, it will be understood that in some example embodiments an organic sensor may include one or more photo-sensing devices and may omit one or more color filters 70a, 70b overlapping the one or more photo-sensing devices in the direction extending perpendicular to the top surface 110a. Such one or more photo-sensing devices may be configured to sense light having a particular, limited wavelength spectrum of light in the absence of the light being filtered by a color filter prior to being received at the photo-sensing device. Accordingly, it will be understood that, in some example embodiments, the organic sensors 500, 600, 700, 800, 900, 1000, and 1800 may omit the color filters illustrated in said organic sensors.

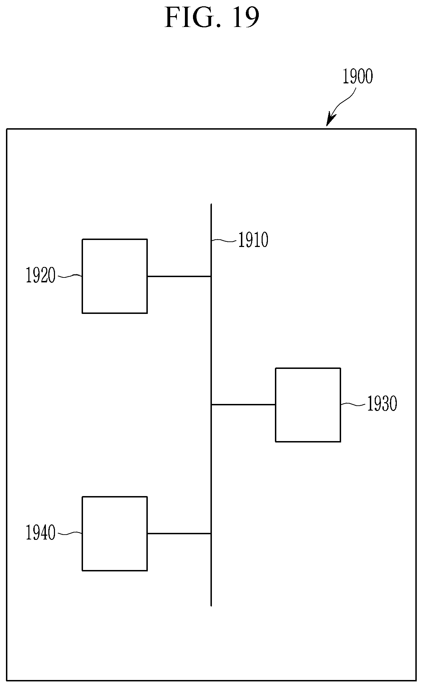

FIG. 19 is a schematic diagram of an electronic device 1900 according to some example embodiments.

As shown in FIG. 19, an electronic device 1900 may include a processor 1920, a memory 1930, and an organic sensor 1940 that are electrically coupled together via a bus 1910. The organic sensor 1940 may be an organic sensor of any of the example embodiments as described herein, and the organic sensor included in the organic sensor 1940 may include any of the photoelectric conversion devices described herein according to any of the example embodiments of the inventive concepts. The memory 1930, which may be a non-transitory computer readable medium, may store a program of instructions. The processor 1920 may execute the stored program of instructions to perform one or more functions. In some example embodiments, the processor 1920 may be configured to process electric signals generated by the organic sensor 1940. The processor 1920 may be configured to generate an output (e.g., an image to be displayed on a display interface) based on processing the electric signals.

Hereinafter, some reference examples are illustrated in more detail.

### Reference Example 1

A 150 nm-thick anode is formed by sputtering ITO on a glass substrate.

Subsequently, a compound represented by Chemical Formula A is deposited on the anode to provide a 5 nm-thick charge blocking layer. Subsequently, a p-type semiconductor (λₘₐₓ: 545 nm, charge mobility: 1.5x10⁻⁵ cm²/Vs) represented by Chemical Formula B-1 and a n-type semiconductor that is fullerene (C60) are codeposited on the charge blocking layer in a volume ratio of 1:1 to provide a 90 nm-thick photoelectric conversion layer. Subsequently, an organic semiconductor (charge mobility: 1.8x10⁻² cm²/Vs) represented by Chemical Formula C-1 is thermally evaporated on the photoelectric conversion layer to provide a 5 nm-thick organic auxiliary layer. Subsequently, ITO (WF: 4.7 eV) is sputtered on the organic auxiliary layer to provide a 7 nm-thick cathode. Subsequently, aluminum oxide (Al₂O₃) is deposited on the cathode to provide a 50 nm-thick anti-reflection layer and sealed with a glass plate to provide a photoelectric conversion device.

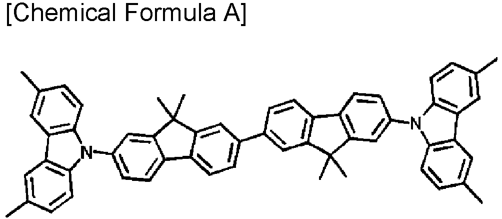

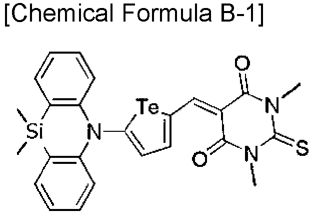

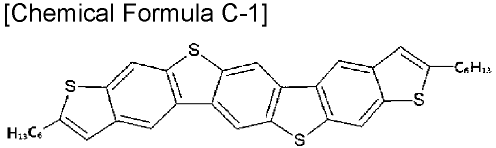

Each charge mobility of the p-type semiconductor represented by Chemical Formula B-1 and the organic semiconductor represented by Chemical Formula C-1 is measured by a time-of-flight. The time-of-flight is a method in which the charge mobility is specifically measured by forming a 1 µm-thick semiconductor layer including the p-type semiconductor represented by Chemical Formula B-1 or the organic semiconductor represented by Chemical Formula C-1 between the ITO electrode and the Al electrode (80 nm), and evaluating a light current.

### Reference Example 2

A photoelectric conversion device is manufactured in accordance with the same procedure as in Reference Example 1, except that Yb (WF: 2.6 eV) is thermally evaporated on the organic auxiliary layer to additionally provide a 1.5 µm-thick inorganic nanolayer.

### Comparative Example 1

A photoelectric conversion device is manufactured in accordance with the procedure as in Reference Example 1, except that the organic auxiliary layer is not formed.

### Reference Example 3

A photoelectric conversion device is manufactured in accordance with the same procedure as in Reference Example 1, except that the organic auxiliary layer is not formed, and a 100 nm-thick photoelectric conversion layer is formed instead of the 90 nm-thick photoelectric conversion layer.

### Reference Evaluation I

A photoelectric conversion efficiency of each photoelectric conversion device according to the Comparative Example and Reference Examples is evaluated according to a wavelength.

The photoelectric conversion efficiency may be evaluated from an external quantum efficiency (EQE) at a maximum absorption wavelength (λₘₐₓ) (about 540 nm) and evaluated in a wavelength spectrum of light of 400 nm to 720 nm by Incident Photon to Current Efficiency (IPCE) method.

The results are shown in Table 1.

**(Table 1)**

| | EQE (@λₘₐₓ, 3 V) (%) |
|---|---|
| Reference Example 1 | 68.0 |
| Reference Example 2 | 69.4 |
| Comparative Example 1 | 66.2 |
| Reference Example 3 | 69.2 |

Referring to Table 1, it is confirmed that the photoelectric conversion devices according to Reference Examples 1 and 2 improve the photoelectric conversion efficiency compared with the photoelectric conversion device according to Comparative Example 1.

In addition, it is confirmed that the photoelectric conversion devices according to Reference Examples 1 and 2 show an equivalent level to photoelectric conversion efficiency to the photoelectric conversion device according to Reference Example 3 having a thick photoelectric conversion layer, thereby, it is also confirmed that the photoelectric conversion devices according to Reference Examples 1 and 2 show a high photoelectric conversion efficiency while reducing the thickness of the photoelectric conversion layer.

### Reference Evaluation II

The photoelectric conversion efficiency of each photoelectric conversion device according to Reference Examples 1 and 2 and Comparative Example 1 is evaluated depending upon an applied voltage.

The results are shown in Table 2.

**(Table 2)**

| | Photoelectric conversion efficiency (EQE, %) | | |
|---|---|---|---|
| | @ 0V | @ 2V | @ 7V |
| Reference Example 1 | 52.0 | 65.1 | 74.8 |
| Reference Example 2 | 55.7 | 66.8 | 75.6 |
| Comparative Example 1 | 49.8 | 63.2 | 72.7 |

Referring to Table 2, it is confirmed that the photoelectric conversion devices according to Reference Examples 1 and 2 improve photoelectric conversion efficiency at each applying voltage compared with the photoelectric conversion device according to Comparative Example 1.

### Reference Evaluation III

Image lag characteristics are evaluated for photoelectric conversion devices according to Reference Examples 1 and 2 and Comparative Example 1.

The image lag may be evaluated by irradiating a light in a wavelength spectrum of light in which a photoelectric conversion may occur into the photoelectric conversion devices according to Reference Examples and Comparative Examples for a particular (or, alternatively, predetermined) time (for example, 33 miliseconds); turning off the light; and measuring a time to take for reaching 6 pA by using a Keithley 2400 equipment. As the image lag is the higher, the electron remains the more, thus after-images may be much frequently found.

The results are shown in Table 3.

**(Table 3)**

| | Lag (msec) |
|---|---|
| Reference Example 1 | 49 |
| Reference Example 2 | 46 |
| Comparative Example 1 | 53 |

Referring to Table 3, it is confirmed that the photoelectric conversion devices according to Reference Examples 1 and 2 show reduced image lag compared with the photoelectric conversion device according to Comparative Example 1, thereby it is estimated that the charge extract characteristic is improved.

### Reference Evaluation IV

Organic sensors employing the photoelectric conversion devices according to Reference Examples 1 and 2 and Comparative Example 1 are designed, and the organic sensor is evaluated for YSNR10.

The YSNR10 of the organic sensor is a minimum light dose (unit: lux) in which a ratio of signal to noise (signal/noise) becomes 10, wherein the signal is a signal sensitivity obtained by performing a RGB raw signal calculated by a FDTD (finite difference time domain method) with a color correction step through a color correction matrix (CCM), and the noise is a noise generated when measuring the signal in the organic sensor. The color correction step is a step of reducing a difference from the real color by image-processing the RGB raw signal obtained from the organic sensor. As the YSNR10 has the lower value, the image characteristics are getting the better in a low light dose.

The results are shown in Table 4.

**(Table 4)**

| | YSNR10 |
|---|---|
| Reference Example 1 | 85 |
| Reference Example 2 | 84 |
| Comparative Example 1 | 86 |

Referring to Table 4, it is confirmed that the photoelectric conversion devices according to Reference Examples show lower YSNR10 than the photoelectric conversion device according to Comparative Example, thereby it is estimated that a sensitivity of the organic sensor may be improved.

## Claims

1. A photoelectric conversion device (100, 200, 300, 400, 100a, 100b, 100c), comprising:
a first electrode (10, 10a, 10b, 10c) and a second electrode (20, 20a, 20b, 20c) facing each other, wherein the first electrode is a cathode and the second electrode is an anode;
a photoelectric conversion layer (30, 30a, 30b, 30c) between the first electrode and the second electrode, the photoelectric conversion layer configured to absorb light in at least one part of a wavelength spectrum of light and to convert the absorbed light into an electric signal, the photoelectric conversion layer including:
a first organic material which is a p-type semiconductor represented by Chemical Formula 1A and
an n-type semiconductor material that forms a pn junction with the first organic material,
wherein the photoelectric layer is an intrinsic layer, and the first organic material and the n-type semiconductor material are mixed as a bulk heterojunction;
and
an organic auxiliary layer (40 40a, 40b, 40c) between the first electrode and the photoelectric conversion layer, the organic auxiliary layer having a higher electron mobility than the electron mobility of the photoelectric conversion layer, the organic auxiliary layer including a second organic material and the n-type semiconductor material, the second organic material being different from the first organic material, and the second organic material and the n-type semiconductor material are mixed,
wherein the electron mobility of the second organic material is 100 times as high as the electron mobility of the first organic material or more than 100 times greater than the electron mobility of the first organic material, and wherein the electron mobility is measured using a time-of-flight method as detailed in the description,
**characterized in that** the second organic material is represented by Chemical Formula 2A or 2B 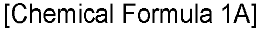 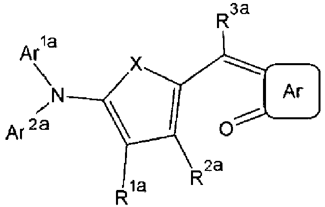
In Chemical Formula 1A,
X is S, Se, Te, SO, SO₂, or SiR^{a}R^{b},
Ar is a substituted or unsubstituted C6 to C30 arylene group, a substituted or unsubstituted C3 to C30 heterocyclic group, or a fused ring of the foregoing two or more,
Ar^{1a} and Ar^{2a} are independently a substituted or unsubstituted C6 to C30 aryl group or a substituted or unsubstituted C3 to C30 heteroaryl group,
Ar^{1a} and Ar^{2a} are independently present alone or may be linked with each other to form a fused ring, and
R^{1a} to R^{3a}, R^{a}, and R^{b} are independently hydrogen, deuterium, a substituted or unsubstituted C1 to C30 alkyl group, a substituted or unsubstituted C6 to C30 aryl group, a substituted or unsubstituted C3 to C30 heteroaryl group, a substituted or unsubstituted C1 to C6 alkoxy group, a halogen, or a cyano group, 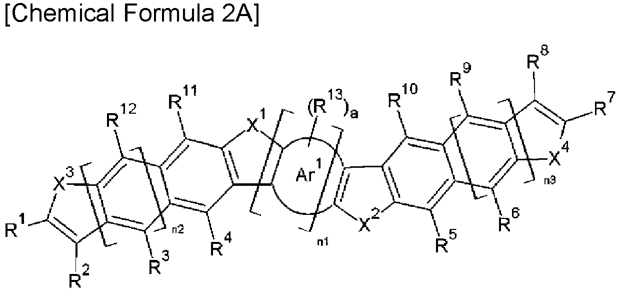 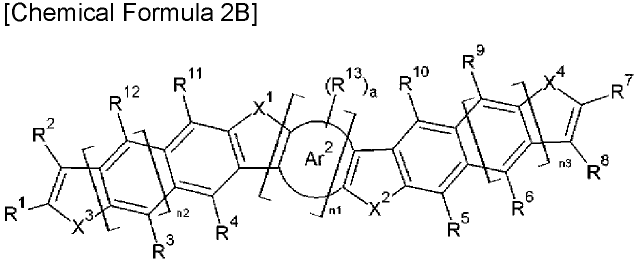
In Chemical Formulae 2A and 2B,
Ar¹ and Ar² are independently a substituted or unsubstituted benzene, a substituted or unsubstituted naphthalene, or a substituted or unsubstituted anthracene,
a corresponds to the number of hydrogen bound to carbon of Ar¹ and Ar²,
X¹ to X⁴ are independently O, S, Se, Te, or N-R^{a}, wherein R^{a} may independently be hydrogen, a substituted or unsubstituted C1 to C30 alkyl group, a substituted or unsubstituted C2 to C30 alkenyl group, a substituted or unsubstituted C2 to C30 alkynyl group, a substituted or unsubstituted C7 to C30 arylalkyl group, a substituted or unsubstituted C6 to C30 aryl group, a substituted or unsubstituted C1 to C30 alkoxy group, a substituted or unsubstituted C6 to C30 aryloxy group (-OR^{b}, wherein R^{b} is a substituted or unsubstituted C6 to C30 aryl group), a substituted or unsubstituted C4 to C30 cycloalkyl group, a substituted or unsubstituted C4 to C30 cycloalkyloxy group (-OR^{c}, wherein R^{c} is a substituted or unsubstituted C4 to C30 cycloalkyl group), a substituted or unsubstituted C2 to C30 heteroaryl group, acyl group (-C(=O)R^{d}, wherein R^{d} is a substituted or unsubstituted C1 to C30 alkyl group), a sulfonyl group (-S(=O)₂R^{e}, wherein R^{e} is a substituted or unsubstituted C1 to C30 alkyl group), or carbamate group (-NHC(=O)OR^{f}, wherein R^{f} is a substituted or unsubstituted C1 to C30 alkyl group),
R¹ to R¹³ are independently hydrogen, a substituted or unsubstituted C1 to C30 alkyl group, a substituted or unsubstituted C1 to C30 alkoxy group, a substituted or unsubstituted C2 to C30 alkenyl group, a substituted or unsubstituted C2 to C30 alkynyl group, a substituted or unsubstituted C6 to C30 aryl group, a substituted or unsubstituted C2 to C30 heteroaryl group, a substituted or unsubstituted C7 to C30 arylalkyl group, a substituted or unsubstituted C2 to C30 heteroarylalkyl group, a substituted or unsubstituted C2 to C30 alkylheteroaryl group, a substituted or unsubstituted C5 to C30 cycloalkyl group, or a substituted or unsubstituted C2 to C30 heterocycloalkyl group,
n1 is 0 or 1,
n2 and n3 are independently 0, 1, 2, or 3,
when n1 is 0, n2 and n3 are 1, 2, or 3, and
when n1 is 1, n1+n2+n3≥2.

2. The photoelectric conversion device of claim 1, wherein the electron mobility of the second organic material is greater than or equal to 1.0x10⁻³ cm²/Vs.

3. The photoelectric conversion device of claims 1 or 2, wherein the n-type semiconductor material includes fullerene or a fullerene derivative.

4. The photoelectric conversion device of any of claims 1-3, wherein a thickness of the organic auxiliary layer is less than or equal to 5 nm.

5. The photoelectric conversion device of any of claims 1-4, further comprising:
an inorganic nanolayer (45) between the first electrode and the photoelectric conversion layer.

6. The photoelectric conversion device of claim 5, wherein the inorganic nanolayer includes a lanthanide element, calcium (Ca), potassium (K), aluminum (Al), or an alloy thereof;
preferably wherein the inorganic nanolayer includes the lanthanide element, and the lanthanide element includes ytterbium (Yb).

7. The photoelectric conversion device of claims 5 or 6, wherein a thickness of the inorganic nanolayer is less than or equal to 5 nm; and/or
wherein the organic auxiliary layer is in contact with the photoelectric conversion layer and the inorganic nanolayer is in contact with the first electrode.

8. An electronic device (1900) comprising the photoelectric conversion device of any of claims 1-7.

9. An organic sensor (1940) comprising the photoelectric conversion device of any of claims 1-7.

10. An electronic device comprising the organic sensor of claim 9.

## Patentansprüche

1. Photoelektrische Umwandlungsvorrichtung (100, 200, 300, 400, 100a, 100b, 100c), umfassend:
eine erste Elektrode (10, 10a, 10b, 10c) und eine zweite Elektrode (20, 20a, 20b, 20c), die einander zugewandt sind, wobei die erste Elektrode eine Kathode ist und die zweite Elektrode eine Anode ist;
eine photoelektrische Umwandlungsschicht (30, 30a, 30b, 30c) zwischen der ersten Elektrode und der zweiten Elektrode, wobei die photoelektrische Umwandlungsschicht dazu konfiguriert ist, Licht in zumindest einem Teil eines Wellenlängenspektrums von Licht zu absorbieren und das absorbierte Licht in ein elektrisches Signal umzuwandeln, wobei die photoelektrische Umwandlungsschicht Folgendes beinhaltet:
ein erstes organisches Material, das ein p-Typ-Halbleiter dargestellt durch die chemische Formel 1A ist, und
ein n-Typ-Halbleitermaterial, das einen pn-Übergang mit dem ersten organischen Material bildet,
wobei die photoelektrische Schicht eine intrinsische Schicht ist und das erste organische Material und das n-Typ-Halbleitermaterial als Bulk-Heteroübergang gemischt sind; und
eine organische Hilfsschicht (40, 40a, 40b, 40c) zwischen der ersten Elektrode und der photoelektrischen Umwandlungsschicht, wobei die organische Hilfsschicht eine höhere Elektronenmobilität als die Elektronenmobilität der photoelektrischen Umwandlungsschicht aufweist, wobei die organische Hilfsschicht ein zweites organisches Material und das n-Typ-Halbleitermaterial beinhaltet, wobei das zweite organische Material von dem ersten organischen Material verschieden ist und das zweite organische Material und das n-Typ-Halbleitermaterial gemischt sind,
wobei die Elektronenmobilität des zweiten organischen Materials 100-mal so hoch wie die Elektronenmobilität des ersten organischen Materials oder mehr als 100-mal größer als die Elektronenmobilität des ersten organischen Materials ist, und wobei die Elektronenmobilität unter Verwendung eines Flugzeitverfahrens, wie in der Beschreibung detailliert, gemessen wird,
**dadurch gekennzeichnet, dass** das zweite organische Material durch die chemische Formel 2A oder 2B dargestellt ist, 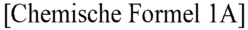 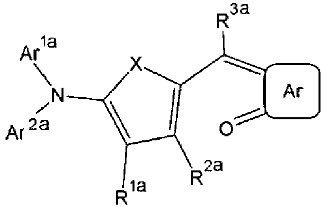
wobei in der chemischen Formel 1A
X S, Se, Te, SO, SO₂ oder SiR^{a}R^{b} ist,
Ar eine substituierte oder unsubstituierte C6- bis C30-Arylengruppe, eine substituierte oder unsubstituierte heterocyclische C3- bis C30-Gruppe oder ein kondensierter Ring der vorstehenden zwei oder mehr ist,
Ar^{1a} und Ar^{2a} unabhängig eine substituierte oder unsubstituierte C6- bis C30-Arylgruppe oder eine substituierte oder unsubstituierte C3- bis C30-Heteroarylgruppe sind,
Ar^{1a} und Ar^{2a} unabhängig allein vorhanden sind oder miteinander verknüpft sein können, um einen kondensierten Ring zu bilden, und
R^{1a} bis R^{3a}, R^{a} und R^{b} unabhängig Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C3- bis C30-Heteroarylgruppe, eine substituierte oder unsubstituierte C1- bis C6-Alkoxygruppe, ein Halogen oder eine Cyanogruppe sind, 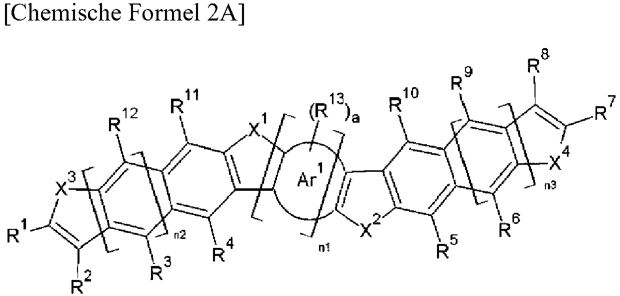 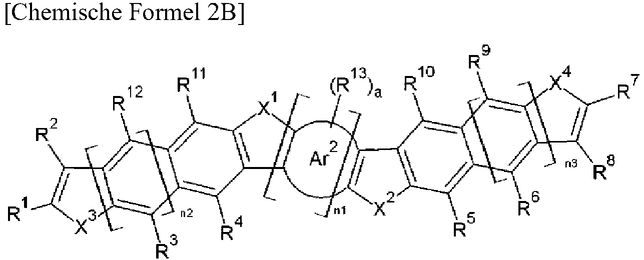
wobei in der chemischen Formel 2A und 2B
Ar¹ und Ar² unabhängig ein substituiertes oder unsubstituiertes Benzol, ein substituiertes oder unsubstituiertes Naphthalen oder ein substituiertes oder unsubstituiertes Anthracen sind,
a der Anzahl an Wasserstoff gebunden an Kohlenstoff von Ar¹ und Ar² entspricht,
X¹ bis X⁴ unabhängig O, S, Se, Te oder N-R^{a} sind, wobei R^{a} unabhängig Wasserstoff, eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkinylgruppe, eine substituierte oder unsubstituierte C7- bis C30-Arylalkylgruppe, eine substituierte oder unsubstituierte C6- bis C30-Arylgruppe, eine substituierte oder unsubstituierte C1- bis C30-Alkoxygruppe, eine substituierte oder unsubstituierte C6- bis C30-Aryloxygruppe (-OR^{b}, wobei R^{b} eine substituierte oder unsubstituierte C6- bis C30-Arylgruppe ist), eine substituierte oder unsubstituierte C4- bis C30-Cycloalkylgruppe, eine substituierte oder unsubstituierte C4- bis C30-Cycloalkyloxygruppe (-OR^{c}, wobei R^{c} eine substituierte oder unsubstituierte C4- bis C30-Cycloalkylgruppe ist), eine substituierte oder unsubstituierte C2- bis C30-Heteroarylgruppe, Acylgruppe (-C(=O)R^{d}, wobei R^{d} eine substituierte oder unsubstituierte C1- bis C30-Alkylgruppe ist), eine Sulfonylgruppe (-S(=O)₂R^{e}, wobei R^{e} eine substituierte oder unsubstituierte C1- bis C30-Alkylgruppe ist) oder Carbamatgruppe (-NHC(=O)OR^{f}, wobei R^{f} eine substituierte oder unsubstituierte C1- bis C30-Alkylgruppe ist) ist,
R¹ bis R¹³ unabhängig Wasserstoff, eine substituierte oder unsubstituierte C1- bis C30-Alkylgruppe, eine substituierte oder unsubstituierte C1- bis C30-Alkoxygruppe, eine substituierte oder unsubstituierte C2- bis C30-Alkenylgruppe, eine substituierte oder unsubstituierte C2- bis C30-Alkinylgruppe, eine substituierte oder unsubstituierte C6- bis C30-Arylgruppe, eine substituierte oder unsubstituierte C2- bis C30-Heteroarylgruppe, eine substituierte oder unsubstituierte C7- bis C30-Arylalkylgruppe, eine substituierte oder unsubstituierte C2- bis C30-Heteroarylalkylgruppe, eine substituierte oder unsubstituierte C2- bis C30-Alkylheteroarylgruppe, eine substituierte oder unsubstituierte C5- bis C30-Cycloalkylgruppe oder eine substituierte oder unsubstituierte C2- bis C30-Heterocycloalkylgruppe sind,
n1 0 oder 1 ist,
n2 und n3 unabhängig 0, 1, 2 oder 3 sind,
wenn n1 0 ist, n2 und n3 1, 2 oder 3 sind, und
wenn n1 1 ist, n1+n2+n3≥2.

2. Photoelektrische Umwandlungsvorrichtung nach Anspruch 1, wobei die Elektronenmobilität des zweiten organischen Materials größer als oder gleich 1,0x10⁻³ cm²/Vs ist.

3. Photoelektrische Umwandlungsvorrichtung nach Anspruch 1 oder 2, wobei das n-Typ-Halbleitermaterial Fulleren oder ein Fulleren-Derivat beinhaltet.

4. Photoelektrische Umwandlungsvorrichtung nach einem der Ansprüche 1-3, wobei eine Dicke der organischen Hilfsschicht weniger als oder gleich 5 nm ist.

5. Photoelektrische Umwandlungsvorrichtung nach einem der Ansprüche 1-4, ferner umfassend:
eine anorganische Nanoschicht (45) zwischen der ersten Elektrode und der photoelektrischen Umwandlungsschicht.

6. Photoelektrische Umwandlungsvorrichtung nach Anspruch 5, wobei die anorganische Nanoschicht ein Lanthanidelement, Calcium (Ca), Kalium (K), Aluminium (Al) oder eine Legierung davon beinhaltet;
wobei bevorzugt die anorganische Nanoschicht das Lanthanidelement beinhaltet und das Lanthanidelement Ytterbium (Yb) beinhaltet.

7. Photoelektrische Umwandlungsvorrichtung nach Anspruch 5 oder 6, wobei eine Dicke der anorganischen Nanoschicht weniger als oder gleich 5 nm ist; und/oder
wobei die organische Hilfsschicht in Kontakt mit der photoelektrischen Umwandlungsschicht ist und die anorganische Nanoschicht in Kontakt mit der ersten Elektrode ist.

8. Elektronische Vorrichtung (1900), umfassend die photoelektrische Umwandlungsvorrichtung nach einem der Ansprüche 1-7.

9. Organischer Sensor (1940), umfassend die photoelektrische Umwandlungsvorrichtung nach einem der Ansprüche 1-7.

10. Elektronische Vorrichtung, umfassend den organischen Sensor nach Anspruch 9.

## Revendications

1. Dispositif de conversion photoélectrique (100, 200, 300, 400, 100a, 100b, 100c), comprenant :
une première électrode (10, 10a, 10b, 10c) et une seconde électrode (20, 20a, 20b, 20c) se faisant face l'une l'autre, dans lequel la première électrode est une cathode et la seconde électrode est une anode ;
une couche de conversion photoélectrique (30, 30a, 30b, 30c) entre la première électrode et la seconde électrode, la couche de conversion photoélectrique étant configurée pour absorber la lumière dans au moins une partie d'un spectre de longueur d'onde de la lumière et pour convertir la lumière absorbée en un signal électrique, la couche de conversion photoélectrique comprenant :
un premier matériau organique qui est un semi-conducteur de type p représenté par la formule chimique 1A et
un matériau semi-conducteur de type n qui forme une jonction pn avec le premier matériau organique,
dans lequel la couche photoélectrique est une couche intrinsèque, et le premier matériau organique et le matériau semi-conducteur de type n sont mélangés en tant qu'hétérojonction en volume ; et
une couche auxiliaire organique (40 40a, 40b, 40c) entre la première électrode et la couche de conversion photoélectrique, la couche auxiliaire organique ayant une mobilité électronique supérieure à la mobilité électronique de la couche de conversion photoélectrique, la couche auxiliaire organique comprenant un second matériau organique et le matériau semi-conducteur de type n, le second matériau organique étant différent du premier matériau organique, et le second matériau organique et le matériau semi-conducteur de type n sont mélangés,
dans lequel la mobilité électronique du second matériau organique est 100 fois supérieure à la mobilité électronique du premier matériau organique ou plus de 100 fois supérieure à la mobilité électronique du premier matériau organique,
et dans lequel la mobilité électronique est mesurée à l'aide d'un procédé de temps de vol tel que détaillé dans la description,
**caractérisé en ce que** le second matériau organique est représenté par la formule chimique 2A ou 2B 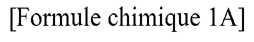 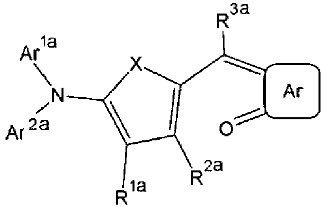
dans la formule chimique 1A,
X est S, Se, Te, SO, SO₂ ou SiR^{a}R^{b},
Ar est un groupe arylène en C6 à C30 substitué ou non substitué, un groupe hétérocyclique en C3 à C30 substitué ou non substitué ou un cycle fusionné des deux précédents ou plus,
Ar^{1a} et Ar^{2a} sont indépendamment un groupe aryle en C6 à C30 substitué ou non substitué ou un groupe hétéroaryle en C3 à C30 substitué ou non substitué,
Ar^{1a} et Ar^{2a} sont présents seuls indépendamment ou peuvent être liés l'un à l'autre pour former un cycle fusionné, et
R^{1a} à R^{3a}, R^{a} et R^{b} sont indépendamment l'hydrogène, le deutérium, un groupe alkyle en C1 à C30 substitué ou non substitué, un groupe aryle en C6 à C30 substitué ou non substitué, un groupe hétéroaryle en C3 à C30 substitué ou non substitué, un groupe alcoxy en C1 à C6 substitué ou non substitué, un halogène, un groupe cyano, 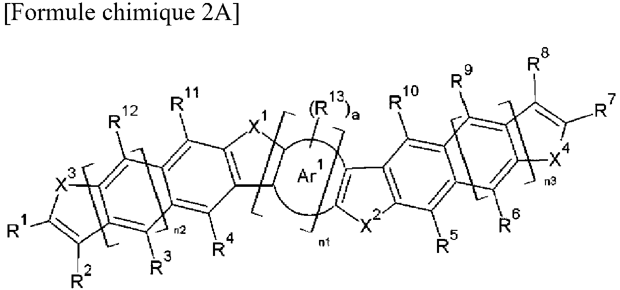 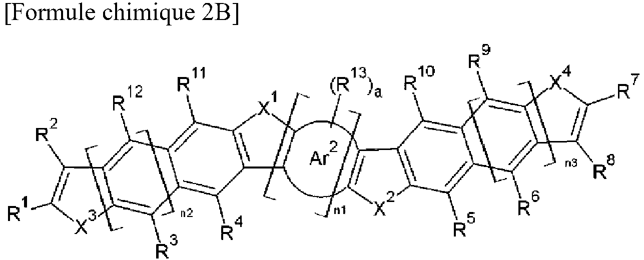
dans les Formules Chimiques 2A et 2B,
Ar¹ et Ar² sont indépendamment un benzène substitué ou non substitué, un naphtalène substitué ou non substitué, ou un anthracène substitué ou non substitué,
a correspond au nombre d'hydrogène lié au carbone de Ar¹ et Ar²,
X¹ à X⁴ sont indépendamment O, S, Se, Te ou N-R^{a}, dans lequel R^{a} peut être indépendamment l'hydrogène, un groupe alkyle en C1 à C30 substitué ou non substitué, un groupe alcényle en C2 à C30 substitué ou non substitué, un groupe alcynyle en C2 à C30 substitué ou non substitué, un groupe arylalkyle en C7 à C30 substitué ou non substitué, un groupe aryle en C6 à C30 substitué ou non substitué, un groupe alcoxy en C1 à C30 substitué ou non substitué, un groupe aryloxy en C6 à C30 substitué ou non substitué (-OR^{b}, dans lequel R^{b} est un groupe aryle en C6 à C30 substitué ou non substitué), un groupe cycloalkyle en C4 à C30 substitué ou non substitué, un groupe cycloalkyloxy en C4 à C30 substitué ou non substitué (-OR^{c}, dans lequel R^{c} est un groupe cycloalkyle en C4 à C30 substitué ou non substitué), un groupe hétéroaryle en C2 à C30 substitué ou non substitué, un groupe acyle (-C(=O)R^{d}, dans lequel R^{d} est un groupe alkyle en C1 à C30 substitué ou non substitué), un groupe sulfonyle (-S(=O)₂R^{e}, dans lequel R^{e} est un groupe alkyle en C1 à C30 substitué ou non substitué), ou un groupe carbamate (-NHC(=O)OR^{f}, dans lequel R^{f} est un groupe alkyle en C1 à C30 substitué ou non substitué),
R¹ à R¹³ sont indépendamment l'hydrogène, un groupe alkyle en C1 à C30 substitué ou non substitué, un groupe alcoxy en C1 à C30 substitué ou non substitué, un groupe alcényle en C2 à C30 substitué ou non substitué, un groupe alcynyle en C2 à C30 substitué ou non substitué, un groupe aryle en C6 à C30 substitué ou non substitué, un groupe hétéroaryle en C2 à C30 substitué ou non substitué, un groupe arylalkyle en C7 à C30 substitué ou non substitué, un groupe hétéroarylalkyle en C2 à C30 substitué ou non substitué, un groupe alkylhétéroaryle en C2 à C30 substitué ou non substitué, un groupe cycloalkyle en C5 à C30 substitué ou non substitué, ou un groupe hétérocycloalkyle en C2 à C30 substitué ou non substitué,
n1 est 0 ou 1,
n2 et n3 sont indépendamment 0, 1, 2, ou 3,
lorsque n1 est 0, n2 et n3 sont 1, 2 ou 3, et
lorsque n1 est 1, n1+n2+n3≥2.

2. Dispositif de conversion photoélectrique selon la revendication 1, dans lequel la mobilité électronique du second matériau organique est supérieure ou égale à 1,0 x 10⁻³ cm²/Vs.

3. Dispositif de conversion photoélectrique selon les revendications 1 ou 2, dans lequel le matériau semi-conducteur de type n comprend du fullerène ou un dérivé de fullerène.

4. Dispositif de conversion photoélectrique selon l'une quelconque des revendications 1 à 3, dans lequel une épaisseur de la couche auxiliaire organique est inférieure ou égale à 5 nm.

5. Dispositif de conversion photoélectrique selon l'une quelconque des revendications 1 à 4, comprenant en outre :
une nanocouche inorganique (45) entre la première électrode et la couche de conversion photoélectrique.

6. Dispositif de conversion photoélectrique selon la revendication 5, dans lequel la nanocouche inorganique comprend un élément lanthanide, du calcium (Ca), du potassium (K), de l'aluminium (Al), ou un alliage de ceux-ci ;
de préférence dans lequel la nanocouche inorganique comprend l'élément de lanthanide, et l'élément de lanthanide comprend de l'ytterbium (Yb).

7. Dispositif de conversion photoélectrique selon la revendication 5 ou 6, dans lequel une épaisseur de la nanocouche inorganique est inférieure ou égale à 5 nm ; et/ou
dans lequel la couche auxiliaire organique est en contact avec la couche de conversion photoélectrique et la nanocouche inorganique est en contact avec la première électrode.

8. Dispositif électronique (1900) comprenant le dispositif de conversion photoélectrique selon l'une quelconque des revendications 1 à 7.

9. Capteur organique (1940) comprenant le dispositif de conversion photoélectrique selon l'une quelconque des revendications 1 à 7.

10. Dispositif électronique comprenant le capteur organique selon la revendication 9.
